# EUROPEAN PATENT SPECIFICATION

(11) **EP 4 303 883 B1**
(45) Date of publication and mention of the grant of the patent: **26.08.2026**
(21) Application number: 23195179.9
(22) Date of filing: 10.11.2015
(51) Int. Cl.: G16H 20/10, G16H 40/20

(54) **MANAGEMENT OF MEDICATION PREPARATION WITH DYNAMIC PROCESSING**
VERWALTUNG DER ARZNEIMITTELZUBEREITUNG MIT DYNAMISCHER VERARBEITUNG
GESTION DE LA PRÉPARATION DE MÉDICATION À TRAITEMENT DYNAMIQUE

(30) Priority: 10.11.2014 US 201462077759 P
(43) Date of publication of application: 10.01.2024
(62) Document number(s) of the earlier application(s) in accordance with Art. 76 EPC: 15858745.1 / 3 218 866
(73) Proprietor: Baxter Corporation Englewood, Englewood, CO 80112 (US)
(72) Inventor: PADMANI, Bhavesh S., Hawthorn Woods, 60047 (US); ABBASI, Ghalib A., Englewood, 80112 (US); HIRAVE, Bapu Laxman, Englewood, 80112 (US); CROOKS, Matthew, Englewood, 80112 (US); YASSINE, Maher F., Englewood, 80112 (US)
(74) Representative: Müller-Boré & Partner Patentanwälte PartG mbB

(56) References cited:
- WO-A1-2014/065872
- US-A1- 2003 076 736
- US-A1- 2014 022 569
- US-A1- 2014 156 294
- US-A1- 2014 214 436

## Description

### BACKGROUND

In many medical facilities, medication orders are provided to a pharmacy for preparation of doses corresponding to the medication orders for administration to a patient. In this regard, orders must be entered, received by the pharmacy, validated, and prepared according to manufacturer's specifications or established institutional guidelines. The preparation process involves the selection and, where required, preparation of drug products for administration to patients in compliance with the dose order. Once fulfilled, the resulting drug products (*i.e*., doses) must be delivered to the patient for administration. One environment, by way of example, in which such processes occur is a hospital.

There are points in the foregoing process that may be susceptible to the loss or corruption of information due to miscommunication or other errors. This can be problematic in terms of logging and auditing of medication doses, which may be required by insurance and regulatory requirements. In turn, systems for management of pharmacy work flow have been proposed. For example, U.S. Provisional Patent Application No. 61/975,519 entitled "MANAGEMENT OF MEDICATION DOSE ORDERS" filed on April 4, 2014, which is co-owned with the present application, discloses certain embodiments of a pharmacy workflow management application. Accordingly, at least partially automated pharmacy workflow management applications have been devised to assist in management of the receipt, processing, organization, preparation, verification, and tracking of medication dose orders in the pharmacy or the like. However, further developments would be beneficial to assist in improvements to the pharmacy workflow management application to promote efficiency, reliability, and accuracy related to the preparation and management of medication dose orders.

WO2014065872A1 work stations for use in medical dose preparation management system. A work station may include a camera stand. The camera stand may include a housing enclosing a camera and one or more light sources therein. As such, the camera and light sources may be directed at a medical dose preparation staging region to capture medical dose preparation images of the medical dose preparation staging region. The camera stand may include an adjustable support positionable in a plurality of positions to dispose the camera and light source relative to the medical dose preparation staging region. A base with a removable tray may be provided that include medical receptacle engagement features. The work stations may facilitate improved image quality, efficiency of work flows carried out at the work station, and administrative tasks such as cleaning.

US 2014/022569 A1 describes a method of managing a mixed injection preparation process includes obtaining a mixed injection preparation instruction (e.g., a prescription) and obtaining information about a drug to be potentially included in the mixed injection according to the preparation instruction. The information about the candidate drug is compared to the preparation instruction and a determination as to whether the mixed injection is prepared in accordance with the mixed injection preparation instruction is made based on the information obtained about the candidate drug. A printer is used to print a label indicating whether the mixed injection is prepared in accordance with the mixed injection preparation instruction, and then it is determined whether the label is removed from the printer.

US 2003/076736 A1 describes systems and methods for weighing and charging components of a product using a radio-frequency order picking and inventory control system (ROPICS) are disclosed. A system according to the invention can include a server computer having a back-end database for storing inventory data, a client computer that is communicatively coupled to the server computer via a communications network, and a scale for providing to the client computer a weight signal that represents an actual weight of a component on the scale. The server computer includes a data queue for receiving and managing database transaction requests in real-time, and a transaction server for receiving the database transaction requests from the data queue and for interfacing with back-end database to process the received database transaction requests in real-time.

US 2014/156294 A1 describes a system for preparing and managing patient-specific dose orders includes an order processing server configured to receive the patient-specific dose orders, define a queue of dose orders and distribute the dose orders to at least one dose preparation station. The dose preparation station is in bi-directional communication with the order processing server and has an interface for providing an operator with a protocol associated with each received drug order and specifying a set of steps to fill the drug order. Steps to fill the drug order are captured at the station and a display, positionable independent of the station outputs the dose order queue and metrics concerning activity at the dose preparation station. Systems for preparing patient-specific doses and a method for telepharmacy in which data captured while following the protocol are provided to a remote site for review and approval by a pharmacist.

US 2014/214436 A1 describes a system for component based aggregation of medication orders may include a processor and memory. The processor may receive display a queue that lists medication orders to be prepared by a healthcare professional, the medication orders indicating component medications that will be used to prepare the ordered medications. The processor may receive a selection of a first medication order listed in the queue, where the first medication order indicates a first component medication. The processor may determine a second medication order listed in the queue that indicates the first component medication. The processor may reorder the displayed queue such that the second medication order is listed adjacent to the first medication order. The processor may notify the healthcare professional of the second medication order, such as by displaying an indication of the second medication order separately from the queue.

### SUMMARY

In view of the foregoing, there is provided a system for batch processing of a plurality of dose orders for preparing corresponding compounded medication doses as defined in the claims.

### BRIEF DESCRIPTION OF THE DRAWINGS

Fig. 1 is a schematic view of an embodiment of a system including a pharmacy workflow management application executed at a local system.
Fig. 2 illustrates a flow diagram of an embodiment of a method of batch processing of a plurality of dose orders in a pharmacy workflow management application.
Fig. 3 illustrates a flow diagram of an embodiment of a method for dynamic determination of a final container for use in a dose order preparation protocol of a pharmacy workflow management application.
Fig. 4a illustrates a flow diagram of an embodiment of a method for dynamic determination of a final container for use in a dose order preparation protocol of a pharmacy workflow management application.
Fig. 4b illustrates a flow diagram of an embodiment of a method for a QS volume determination for use in an embodiment of a method for dynamic determination of a final container for use in a dose order preparation protocol of a pharmacy workflow management application.
Fig. 4c illustrates a flow diagram of an embodiment of a method for a QS volume sufficiency check for use in an embodiment of a method for dynamic determination of a final container for use in a dose order preparation protocol of a pharmacy workflow management application.
Fig. 4d illustrates a flow diagram of an embodiment of a method for the identification of active workstation products for use in an embodiment of a method for dynamic determination of a final container for use in a dose order preparation protocol of a pharmacy workflow management application.
Fig. 4e illustrates a flow diagram of an embodiment of a method for dynamic determination of a final container for use in a dose order preparation protocol of a pharmacy workflow management application.
Fig. 5 illustrates a screenshot of a user interface of the pharmacy workflow application, according to an embodiment.
Fig. 6 illustrates a screenshot of a user interface of the pharmacy workflow application, according to an embodiment.
Fig. 7 illustrates a screenshot a user interface of the pharmacy workflow application, according to an embodiment.
Fig. 8 illustrates a flow diagram of an embodiment of a method for dynamic diluent volume accounting in a dose order preparation protocol of a pharmacy workflow management application.
Fig. 8a illustrates a screenshot of a user interface of the pharmacy workflow application, according to an embodiment.
Fig. 8b illustrates a screenshot of a user interface of the pharmacy workflow application, according to an embodiment.
Fig.8c illustrates a screenshot of a user interface of the pharmacy workflow application, according an embodiment.
Fig. 9 illustrates a screenshot of a user interface of the pharmacy workflow application, according to an embodiment.
Fig. 10 illustrates a flow diagram of an embodiment of a method for managing an error regarding a dose order record in a pharmacy workspace queue in a pharmacy management application.
Fig. 11 illustrates a screenshot of a user interface of the pharmacy workflow application, according to an embodiment.
Fig. 12 illustrates a screenshot of a user interface of the pharmacy workflow application, according to an embodiment.
Fig. 13 illustrates a flow diagram of an embodiment of a method for identifying alerts on a dose order for use in an embodiment of a method for managing an error regarding a dose order record in a pharmacy workspace queue in a pharmacy management application.
Fig. 14 illustrates a flow diagram of an embodiment of a method for identifying alerts on user accounts for use in an embodiment of a method for managing an error regarding a dose order record in a pharmacy workspace queue in a pharmacy management application.
Fig. 15 illustrates a flow diagram of an embodiment of a method for identifying alerts scanned products barcodes for use in an embodiment of a method for managing an error regarding a dose order record in a pharmacy workspace queue in a pharmacy management application.
Fig. 16 illustrates a flow diagram of an embodiment of a method for reprocessing dose order for use in an embodiment of a method for managing an error regarding a dose order record in a pharmacy workspace queue in a pharmacy management application.
Fig. 17 illustrates a flow diagram of an embodiment of a method for requeueing dose orders for use in an embodiment of a method for managing an error regarding a dose order record in a pharmacy workspace queue in a pharmacy management application.

### DETAILED DESCRIPTION

The following description is not intended to limit the invention to the forms disclosed herein. Consequently, variations and modifications commensurate with the following teachings, skill and knowledge of the relevant art, are within the scope of the present invention. The embodiments described herein are further intended to explain modes known of practicing the invention and to enable others skilled in the art to utilize the invention in such, or other embodiments and with various modifications required by the particular applications(s) or use(s) of the present invention.

The present disclosure contemplates a pharmacy workflow management application that may facilitate pharmacy workflow management in relation to preparation of one or more doses at a pharmacy, hospital, or other facility at which doses are prepared for administration to a patient. The pharmacy workflow management application may be executed at a local system (also referred to herein as a local node). The local node may correspond to a facility such as a hospital, pharmacy, or other facility capable of preparing a dose for administration to a patient. Pharmacy workflow management provided by the pharmacy workflow management application may include one or more activities within a pharmacy that may include management of a locally stored formulary, processing received medication dose orders at a pharmacy, creating corresponding dose order records for each received dose order, managing (e.g., viewing, sorting, prioritizing, etc.) dose order records, guiding a pharmacy technician in preparing the dose order medication, gathering information regarding the preparation of a dose to fulfill a dose order, reviewing the medication dose order, and/or tracking of medication dose order records in the pharmacy.

The local node may comprise one or more devices that may include hardware and/or software that may execute machine readable instructions stored as machine readable non-transitory data in a memory. The machine readable instructions, when executed by a processor may provide functionality related to the pharmacy workflow management application discussed herein. In this regard, the pharmacy workflow management application may include one or more processors operative to access machine-readable data stored in a storage medium (i.e., a memory) that provide instructions to the one or more processors for execution to provide the functionality described herein.

The local node executing the pharmacy workflow management application may be in operative communication with a central server. The central server may provide support services to the local node to support the pharmacy workflow management application and/or may provide additional services such as data backup, report generation, or access to data sets stored at the central server.

The present disclosure includes a description of an embodiment of dose order processing that may be performed by the pharmacy workflow management application. For instance, the pharmacy workflow management application may facilitate a method of batch processing of a plurality of dose orders. In another embodiment, the pharmacy workflow application may facilitate the dynamic alteration of a final container in a dose order preparation protocol. In yet another embodiment, the pharmacy workflow application may facilitate dynamic diluent volume accounting and/or adjustment in a dose preparation protocol. In yet another embodiment, the pharmacy workflow application may facilitate managing an error regarding a dose order record.

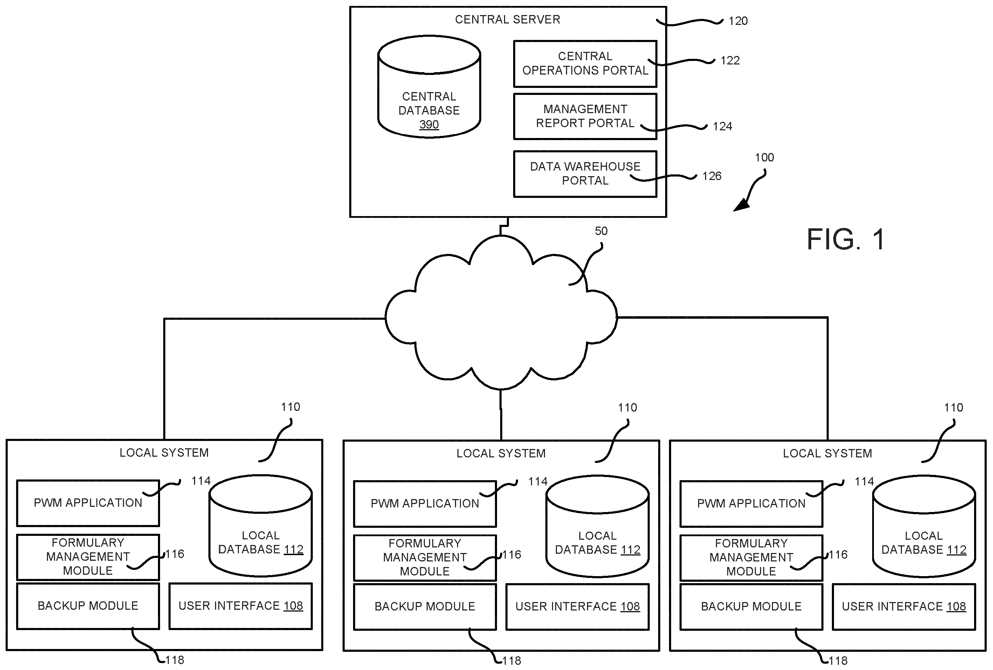

With initial reference to Fig. 1, a system 100 is depicted that includes a plurality of local systems 110. The local systems 110 may each comprise a local node in the system 100. The local systems 110 may each execute a pharmacy workflow management application 114. For instance, each local system 110 may include at least one local device executing a client application for providing functions related to the pharmacy workflow management application. As shown in Fig. 1, a plurality of local systems 110 may be provided that are each in operative communication with a central server 120. For example, the local systems 110 may be in operative communication the central server 120 by way of a wide area network 50 (e.g., the Internet).

Each local system 110 may be a healthcare facility such as a hospital, pharmacy, outpatient clinic, or the like, that prepares doses for administration to a patient. The respective pharmacy workflow management application 114 at each local system 110 may be operative to generate and/or capture medical information that may be provided to the central server 120 by way of the wide area network 50. As will come to be appreciated from the discussion below in relation to the pharmacy workflow management application, the medical information may include medication dose order data that may include metadata regarding the dose order record and/or dose. The pharmacy workflow management application 114 at each local system 110 may allow for the pharmacy workflow management functions described in greater detail below. In any regard, any or all data generated by the pharmacy workflow management application may be provided to the central server 120. In this regard, central server 120 may provide for data collection and/or data backup services in relation to the local systems 110.

Accordingly, in at least one embodiment the local systems 110 may comprise unaffiliated and discrete healthcare facilities capable of preparing medication doses for administration to patients. The central server 120 may be hosted by another discrete and unaffiliated third-party that may be separate from any of entities of the local systems 110. For instance, the central server 120 may be hosted and/or executed by an application provider that provides one or more client applications for execution by the local systems 110 to facilitate the pharmacy workflow management application 114. Specifically, the central server 120 may be executed or hosted by an application provider that provides the pharmacy workflow management application each local system 110.

A local system 110 may include a local database 112 for storage of information. As may be appreciated, the local database 112 may in fact include a collection of one or more databases collectively defining the local database 112. In an implementation of the present invention, the local system 110 may include a server that facilitates access to the database 112. The database may store data related to the current in process work and/or store archival data related to prior work completed at the pharmacy. The local database 112 may be a high-performance, highly scalable and SQL-compliant object database. In this regard, the database may scale easily to handle thousands of simultaneous users and potentially terabytes of data.

In addition to storing information related to pharmacy work (i.e., dose order records), the database 112 may include information in a variety of contexts including information related to formulary information, administrative information, or other information related to the pharmacy workflow management application 114 at the local system 110. In any regard, the local system 110 may execute a number of services (e.g., provided by modules executed by a processor). For instance, a data backup module may be provided that provides a rule-base approach to data backup from the local system 110 to the central server 120. The data backup module may define the interval at which the local system 110 provides backup data to the central server 120 and/or may dictate what information is provided to the central server. The backup module may also provide an administrator (e.g., at the central server or the local system 110) with information relating to the success or failure of data backup operations, system slowdowns, and the like. For instance, the backup module may facilitate selective backup of the local database 112 as described in U.S. Patent Application No. 62/019,227.

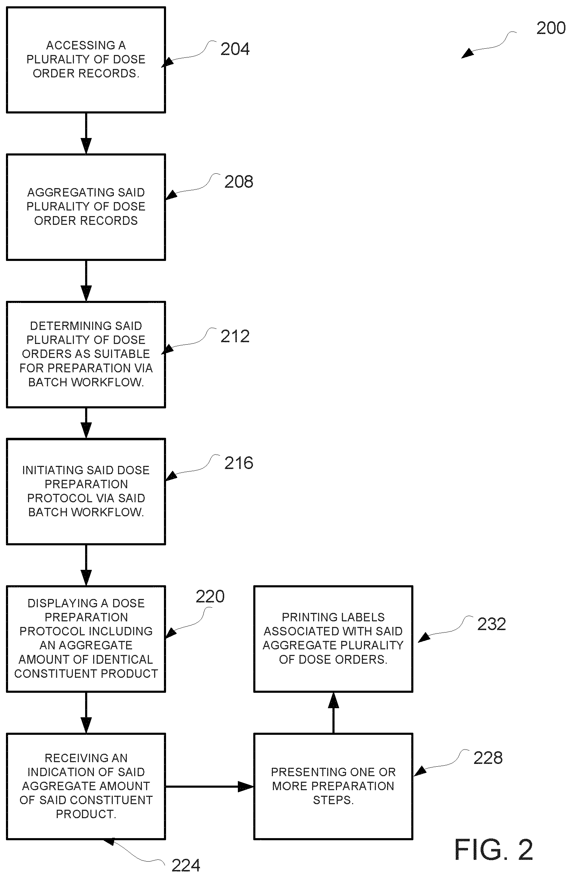

Turning to Fig. 2, in one embodiment, the pharmacy workflow application 114 may facilitate preparation of a plurality of dose orders according to a batch workflow according generally to method 200. In this regard, a plurality of dose orders having identical constituent products associated with different dose order records may be prepared according to a batch workflow such that a plurality of such dose orders may be prepared without initiating a new workflow process for each of the plurality of dose orders.

In some instances, the preparation of dose orders in a batch workflow may facilitate efficient dose order preparation techniques and contribute to overall error reduction related to the dose fulfillment. Due at least in part to the fact that a new workflow is not being initiated for each of the plurality of doses, the batch workflow process may facilitate efficient dose order preparation by potentially eliminating repetitive preparation steps and organizing the steps relative to the plurality of doses such that like steps are performed together. In this regard, the batch workflow may comprise various product identification steps and various dose compounding steps. The product identification steps may establish, based on a received indication of an aggregate amount of at least one constituent product, the presence or possession of a product at, for example, a dose compounding station. The dose compounding steps may include one or more preparation steps regarding the physical preparation of the aggregate plurality of doses. Notably, the batch workflow may execute the various dose compounding steps after the completion of the various product identification steps for each of the aggregate plurality of doses of the batch. As such, the batch workflow may facilitate faster dose order preparation by grouping the product identification steps and the dose compounding steps such that, for example, the presence of at least one constituent product to fulfill the aggregate plurality of dose orders is established prior to the corresponding dose compounding step. For example, as discussed in greater detail below, the batch workflow may be operable to identify an aggregate amount of at least one constituent product of the plurality of doses. In this regard, all the required products *(e.g.,* a diluent, a drug, or another product) may be identified prior to the physical preparation of the plurality of dose orders. This may eliminate repetitive steps related to identifying an amount of a product for each individual dose. The elimination of this and other repetitive steps may save a substantial amount of time, which thereby increases the efficiency of the overall preparation of dose orders. Error rates may also be reduced. For example, each of the plurality of doses prepared via the batch workflow may comprise identical constituent components. In this regard, the products identified during the preparation during the batch workflow may be appropriately associated with any of the plurality of dose orders. This may reduce errors by reducing the occurrence, for example, of inappropriate products being introduced into the prepared dose order.

The pharmacy workflow application 114 may access 204 a plurality of dose order records stored in, for example, local database 112. The dose order records may correspond to dose orders received by the pharmacy workflow application 114 that have not yet been prepared. Furthermore, a dose order may include a request for medication that includes various constituent products. Each dose order accessed 204 by pharmacy workflow application 114 comprising the plurality of dose orders (which may include less then all dose orders at the pharmacy workflow application 114) may include identical constituent products such that each of the plurality of dose orders comprises a request for the same constituent products. In this regard, each dose order may include a plurality of identical data attributes corresponding to, among other things, an identical ingredients listing, identical ingredient amount, identical ingredient concentration, and the like. Accordingly, based in at least in part on the plurality of identical data attributes, the plurality of dose orders may be identified as being eligible for being prepared via the batch workflow, as described in greater detail below.

The pharmacy workflow application 114 may access 204 the plurality of dose orders based at least in part on identifying common associations (e.g., one or more data attributes as described above) among the plurality of dose order records such that each dose order comprises a request for identical constituent products, as described as above. In some embodiments, the common associations of the plurality of dose order records may be identified via a filter applied to the plurality of dose order records. That is, the pharmacy workflow application 114 may access a plurality of dose orders based on the identified common attributes such that the accessed dose order constitute dose orders that include identical constituent products. In some instances, the pharmacy workflow application 114 may access the plurality of dose orders that include identical constituent products from a database of dose orders that include a second plurality of dose orders that do not include identical constituent products as the identical constituent products of the identified plurality. In this regard, the filter may facilitate accessing or identifying dose orders appropriate for preparation via the batch workflow by accessing the plurality of dose orders that include identical constituent products.

The pharmacy workflow application 114 may aggregate 208 the accessed dose order records to create an aggregated plurality of dose orders *(e.g.,* a batch) to facilitate the batch workflow. This may be accomplished in terms of a queue such that each of the plurality of dose orders that share the common associations (e.g., identical constituent products) are grouped together to form the aggregated plurality of dose orders *(e.g.,* each identified dose order of "Cefazolin 1 mg in 10 ml NS syringe" may be grouped together in the queue as an aggregated plurality of dose orders to define a batch of orders). The pharmacy workflow application 114 may prepare the aggregate plurality of dose orders in a common batch according to a batch workflow. The batch workflow may include a protocol for preparation of all dose orders in the batch.

In one embodiment, the queue may facilitate batch processing initiation by determining 212 the plurality of dose orders as suitable for batch processing and initiating 216 the dose preparation protocol corresponding to the aggregate plurality of dose orders for preparation via the batch workflow. In this regard, only those dose order records associated with the queue *(i.e.,* the dose order belongs to a batch of dose orders with identical constituent products) may be may be suitable for preparation according to the batch workflow. In this regard, the queue may facilitate the inclusion of only those dose orders with identical constituent products for preparation via the batch workflow. Accordingly, the pharmacy workflow application 114 may initiate 216 a dose preparation protocol corresponding to the aggregate plurality of dose orders for preparation via the batch workflow based at least in part on the presence of a dose order in of the queue. In some instances, the batch workflow is initiated directly from the queue, or the presence of a generated queue within the pharmacy workflow application 114. Initiating of the batch workflow from the queue may also provide a user safeguard, by ensuring that only the plurality of dose orders of identical constituent products may be selected for the batch workflow.

With returned reference to Fig. 1, local system 110 may also include a user interface 108. The user interface 108 may be generally operable to present and receive information associated with the batch workflow per the operation of the pharmacy workflow application 114. For instance, the pharmacy workflow application 114 may display 220 a dose preparation protocol corresponding to the aggregate plurality of dose order records. The dose preparation protocol may include information pertaining to the required aggregate amount of one or more of the identical constituent products needed to prepare each of the aggregated plurality of dose orders. For the sake of illustration, consider the example where the aggregate plurality of dose orders includes 10 dose orders, each for Cefazolin 1,000 mg, 0.9% Sodium Chloride 250 ml in 300 ml. In this illustration, the dose preparation protocol may include information pertaining to the volume of constituent products required to make all 10 dose orders *(i.e.,* 10,000 mg Cefazolin and 2,900 ml of Sodium Chloride). In this regard, the dose preparation protocol includes requirements information pertaining to the preparation of all of the aggregate plurality of dose orders selected for batch preparation in the batch workflow.

The aggregate plurality of dose orders may be prepared by initially receiving 224 an indication related to products to be used to prepare the batch that identify the aggregate amount of at least one of the constituent products. In this regard, the pharmacy workflow management application 114 may receive a user input indicative of the aggregate amount of the at least one constituent products. For example, the pharmacy workflow management application 114 may receive an indication via one or more product scans, NDC numbers, or other input that correspond to an indication of the possession of a product by a user for use in preparing the aggregate plurality of the dose orders. That is, the indication may correspond to the total amount (e.g., volume or mass) of the product required for preparing the aggregate plurality of dose orders. As such, the corresponding product of the indication may include a product volume such that the product volume is at least equal to the aggregate amount of the at least one constituent products. It some instances, the pharmacy workflow application 114 may require receipt of the indication identifying the aggregate amount of the at least one constituent product before presenting to the user one or more preparation steps of the aggregate plurality of dose orders, as discussed in greater detail below. In this regard, a pharmacy technician, for example, may indicate the possession of the at least one constitute product to the pharmacy workflow application 114 before executing the one or more preparation steps.

In some embodiments, the user input may comprise a plurality of indications. For example, each of the plurality of indications may include indications related to different instances of a given product for use in preparing the aggregate plurality of dose orders. Accordingly, in this embodiment, the multiple instances of the given products, collectively, corresponding to the plurality of indications, may be at least equal to the aggregate amount of the at least one constituent product. In this regard, multiple, separate products may be used to prepare the aggregate plurality of dose orders. As the batch workflow may facilitate the preparation of any sized batch, including larger batches of 50, 100, or more, the indication corresponding to multiple separate products may support the preparation of the aggregate volume for larger batches.

For the sake of illustration, consider again the example where the aggregate plurality of dose orders includes 10 dose orders, each for Cefazolin 1,000 mg, 0.9% Sodium Chloride 250 ml in 300 ml. The pharmacy workflow application 114 may display the dose preparation protocol of the dose that includes information pertaining to the required volume of constituent products necessary to make all 10 dose orders *(i.e.,* 10,000 mg Cefazolin and 2,900 ml of Sodium Chloride), including the physical step associated with a user retrieving the products for use in the preparation of the aggregate plurality of dose orders. In turn, the pharmacy workflow application 114 may receive the user input corresponding to an indication identifying the amount of at least one of the constituent products that the user possesses for use in preparing the dose. For example, the user input may pertain to the 10,000 mg of Cefazolin or the 2,900 ml of Sodium Chloride - *i.e.,* the volume of at least one constituent product required to make all 10 dose orders, thus indicating the user has sufficient product to make dose order in the batch. In some instances, the user input may pertain to both the 10,000 mg of Cefazolin and the 2,900 ml of Sodium Chloride - *i.e.,* the volume of each different respective vials of the constituent products required to make all 10 dose orders. In either case, the user input may include a plurality of indications, each of which may correspond to a different instance of the given product such that the plurality of indications corresponds to the aggregate amount of at least one constituent product. For example, the user may retrieve two 5,000 mg vials of Cefazolin. In turn, where the user scans each vial, each of the plurality of indications may include separate indications for two 5,000 mg vials of Cefazolin. In this regard, the user input would include two indications of the 5,000 mg vial of Cefazolin such that the plurality of indication corresponds to the aggregate amount of the identical constituent product *(e.g.,* 2 vials of 5,000 mg Cefazolin equals the 10,000 mg of Cefazolin, the aggregate amount).

The pharmacy workflow application 114 may also present 228 preparation steps to the user via user interface 108. As previously noted, a product preparation steps comprises the physical steps required to prepare a dose *(e.g.,* for the sake of illustration, remove 10 ml diluent volume from 0.9% Sodium Chloride - 300 ml, Acquire 10 ml from Cefazolin, etc.). Specifically, the pharmacy workflow application 114 may present 228 preparation steps to the user upon receipt of the user input indicative of the aggregate amount of the at least one constituent products. In this regard, the pharmacy workflow application 114 may present preparation steps after all products for the aggregate amount of the at least one constituent product have been identified via the corresponding indication. Accordingly, the aggregate plurality of dose orders of the batch workflow may be prepared collectively such that any single dose order of the aggregate plurality of dose orders will not be prepared until the entire aggregate amount of the at least one constituent product is indicated via the corresponding indication. This may facilitate a batch workflow by segmenting the preparation steps such that each preparation step is performed on each of the aggregated plurality of dose orders before proceeding to the next preparation step (e.g., receiving an indication of an aggregate amount of product sufficient to fulfill the aggregate plurality of orders, before physically preparing any of the aggregate plurality of dose orders, etc.).

In some embodiments, the pharmacy workflow application 114 may print 232 a label for each of the aggregate plurality of dose orders. A label may be applied to a prepared dose order and include information associated with the contents of the prepared dose order. Based on the identified common associations of the queue filter, each of the prepared dose orders may contain identical constituent products. In this regard, printing 232 labels for each of the aggregate plurality of dose orders may be incorporated into the batch workflow process in a particular and separate processing segment of the foregoing. This may include, for example, printing the labels for each of the aggregate plurality of dose orders at a common time. For example, the pharmacy workflow application 114 may print the labels after receiving an indication of the aggregate amount of the at least one constituent product. In other instances, the pharmacy workflow application 114 may print the labels after receiving an indication that the one or more steps of the preparation protocol have been completed. Accordingly, the labels may all be applied at a common time as a discrete step of the batch workflow process.

In another embodiment, the batch workflow may facilitate printing work-in-progress ("WIP") labels such that the pharmacy workflow application 114 may print WIP labels after the completion of the one or more preparation steps. A WIP label may be applied, for example, to a partially-used diluent product to indicate the remaining diluent volume, expiration data, and the like. In this regard, to the extent that the preparation of a dose generates a partially-used diluent product, the preparation of dose via batch workflow may require printing a WIP label only after the preparation of the aggregate plurality of dose orders of the batch.

For example, consider a batch comprised of a hypothetical 10 doses of Cefazolin 1,000 mg, 0.9% Sodium Chloride 250ml in 300 ml. A 3,000 ml bag of Sodium Chloride may satisfy the required diluent to prepare the batch *(i.e.,* 3,000 ml > 2,900 ml required to prepare the batch). If the 10 doses of the hypothetical batch were prepared individually, 10 WIP labels may theoretically be printed *(e.g.,* a first WIP label after the first prepared dose indicating 2,700 ml remaining in the Sodium Chloride bag, a second WIP label after the second prepared dose indicating 2,400 ml remaining in the Sodium Chloride bag, etc.). The multiple printing of WIP labels in this regard may contribute to additional processing steps that may result in process inefficiency. However, the same 10 doses prepared as a batch via the batch processing may result in printing only 1 WIP label, for example. That is, after presenting the one or more preparation steps regarding the aggregate plurality of doses, the pharmacy workflow application 114 may print one WIP label indicating 100 ml remaining in the Sodium Chloride bag *(i.e.,* 3,000 ml bag - 2,900 ml required equals 100 ml).

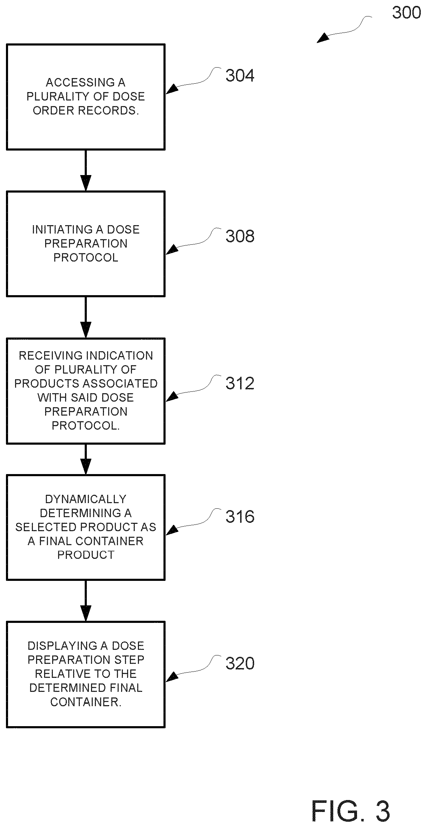

Turning next to Fig. 3, according to another embodiment, the pharmacy workflow application 114 may facilitate the dynamic alteration of a final container in a dose order preparation protocol according generally to method 300. In this regard, a formulary product may be identified as being capable of use as a final container - *i.e.,* the container which will ultimately contain the prepared dose. Accordingly, a dose order need not necessarily be prepared in a new or empty container. Additionally, a user need not guess or make ad hoc choices as to the final container. Rather, the pharmacy workflow application 114 dynamically determines whether a formulary product indicated as being used in a dose preparation protocol is appropriate as a final container based on a received indication of a plurality of products associated with the dose order.

Considered in the context of preparing a dose order, for every dose order preparation there is a final container. The final container may either be a container associated with a formulary product used in the preparation of the dose or some other container (e.g., a new or empty container). Various rules and conditions, explained in greater detail below, executed by the pharmacy workflow application 114 may be applied to a dose order in view of a received indication of a product to be used to prepare the dose in order to dynamically determine a selected product as a final container during the dose preparation protocol. In this regard, based on the rules and conditions, the pharmacy workflow application 114 may receive an indication of a plurality of products to be used to prepare the dose and dynamically determine one of the plurality of products as the final container or whether to prepare a final container. Pharmacy workflow application 114 may also add a visual indicator to a user interface to specify the final container in relation to a listing of products of the dose preparation protocol. After each received indication, the pharmacy workflow application 114 may reevaluate the final container determination decision such that initially identified final container may not be valid until receiving an indication of the plurality of products.

In some instances, the dynamic alteration of a final container may facilitate efficient dose order preparation techniques and contribute to overall error reduction by substantially automating the final container determination. For example, absent the pharmacy workflow application 114 dynamically determining a final container, the user may be required to make a manual determination during the preparation of the dose order. In this regard, previous dose order preparation practices did not automate the final container determination. Rather, previous dose order preparation practice relied substantially on a user's judgment to determine the final container based in part on experiential knowledge. Due in part to the pharmacy workflow application 114 dynamically determining the final container based on a final container characteristic of a selected product, user judgment alone need not be relied upon to complete the determination. In this regard, error rates may be reduced by standardizing the final container determination (e.g., by way of a standard final container algorithm). In particular, dynamically determining the final container based on a final container characteristic of a selected product may reduce errors associated with waste produced during dose order preparation. For example, according to one embodiment, the dose order may only be prepare in a final container where the dose order exceeds a dose order minimum volume *(e.g.,* 20 ml , 24 ml, 20 ml, etc.)

Moreover, dose order preparation efficiency may also be increased because the standardized final container determination may be performed in the same manner in each circumstance. Notably, certain aspects of the standardized final determination may be customizable according to local preferences such as, and discussed in greater detail below, dose order volume rules, waste volume rules, and the like. The customizable aspects may facilitate system-wide consistency as the local preferences may be uniformly applied across a plurality of pharmacy workflow applications 114 operating in a common network, such as wide area network 50, for example. Accordingly, knowing the final container, the pharmacy workflow application 114 may display the correct dose preparation steps associated with preparation of the dose relative to the final container.

With further reference to Fig. 3, which illustrates the dynamic alteration of the final container, which includes the pharmacy workflow application 114 accessing 304 a dose order record corresponding to a dose order stored in, for example, local database 112. The dose order record may correspond to a dose order received by the pharmacy workflow application 114 that has not yet been prepared. The dose order may include a request for medication that is awaiting preparation via the pharmacy workflow application 114. In some instances, the dose order record of the corresponding dose order may be stored in a queue comprised of a plurality of dose order records with corresponding dose orders also awaiting preparation.

A dose preparation protocol may be initiated 308 that corresponds to the accessed dose order. The dose preparation protocol may include a number of product identification steps, which may include list of constituent products or a "recipe" for the dose order. This may include, among other things, a description of each constituent product for preparation of the dose order, the volume or mass required of each constituent product, and the like for use in identify the various products required to prepare the dose order. For example, a dose preparation protocol for a hypothetical dose of Cefazolin 1,000 mg, 0.9% Sodium Chloride 250 ml in 300 ml may identify 1,000 mg of Cefazolin and 250 ml of 0.9% Sodium Chloride as within the dose preparation protocol. Moreover, the dose preparation protocol may include the various dose compounding steps, which may communicate steps associated with the physical preparation of the dose order (e.g., remove diluent, remove drug, apply label to dose, etc.).

The pharmacy workflow application 114 may then receive 312 an indication of a plurality of products associated with the dose preparation protocol. The received indication may be indicative of, for example, a pharmacy technician obtaining possession of the product for use in preparing the dose. In this regard, the pharmacy workflow application 114 receives an indication that a product associated with a formulary data record of the same product has been identified for use in preparing the dose of the dose order. Each corresponding product of the indication may be associated with a plurality of corresponding data attributes per a formulary data record corresponding to the product that may describe, among other items, physical attributes of the product, such as the product volume. In some instances, the plurality of corresponding data attributes may include a data field indicative of the suitability of a product as a final container. In any event, the totality of corresponding data attributes may correspond to a final container characteristic, such that the received indication facilitates a dynamic determination of the final container.

Accordingly, the pharmacy workflow application 114 may dynamically determine 316, in response to the received indication, at least one of the products as a selected product corresponding to a final container based at least in part on analysis of the final container characteristic of the products indicated as being used to prepare the doses. That is, the pharmacy workflow application 114 may determine whether a product associated with the indication that the product is to be used in preparation of the dose order may constitute the final container, which will serve as a destination receptacle to contain the prepared dose. If the container of the indicated formulary product cannot be used as the final container, the dose may be prepared in a new, empty container. The dynamic determination may change for each received indication of the plurality of products associated with the dose preparation protocol during the product identification steps. For example, a first diluent product container may initially be determined to constitute the final container. A second diluent product container indication may be received that modifies the final container determination (e.g., to the second diluent container, or into the empty container) based on receipt of the indicator associated with the second diluent product container. That is, receipt of the indication corresponding to the second diluent container may trigger a change in the final container determination.

The final container characteristic, as noted above, may correspond to a plurality of data attributes associated with the product for use in preparation of the dose order. As such, the dynamic determination may be at least partially based on the execution of a plurality of rules, as discussed in more detail below, operable to determine whether a particular product should constitute the final container (e.g., a volume differential rule may be adopted that dictates that a formulary product should not be a final container if the percentage difference between product total volume and dose order total volume is greater than or equal to a specified differential percentage). The dynamic determination may also be based on characteristics of the dose order itself, such as by requesting a final volume of the order *(e.g.,* the final container may be determined based on an adjusted diluent ingredient volume corresponding to the received product indications).

The previously noted interface 108 of local system 110 may also display 320 a dose preparation step associated with the preparation of the dose relative to the final container. That is, the preparation step of the preparation protocol may be at least partially based on the dynamically determined final container. For instance, the protocol may differ for a given dose order based on the final container determination (e.g., a negative QS volume versus a positive QS volume, etc.). Also, the preparation protocol may include displaying an indication to the user corresponding to the product that constitutes the final container. In some instances, the final container product may be denoted at the user interface with an insignia such as that of the encircled capital letters "FC," for example. As such, the displaying may therefore facilitate communication to the user of which, if any *(i.e.,* in the case of requiring an empty container), container constitutes the final container such that a user may use this information to prepare the dose of the dose order in the noted final container.

The pharmacy workflow application 114 may at least in part facilitate dynamically determining the final container by adjusting the diluent ingredient volume associated with the dose preparation protocol. In particular, the pharmacy workflow application 114 may optimize the dose preparation protocol by adjusting the request diluent ingredient volume before displaying the dose preparation step associated with preparation of the dose order relative to the final container. In order to calculate the exact required diluent volume for the dose order, after indication of a product to be used to prepare the dose *(e.g.,* a product scan), the pharmacy workflow application 114 may identify the currently active workstation products for the dose order in the particular dose order. The pharmacy workflow application 114 may revaluate this identification for each subsequent indication received. The pharmacy workflow application 114 may adjust the diluent ingredient volume, based in part on the volume provided by the drug ingredient and requested final volume of the dose order. The foregoing adjustment may therefore update the requested diluent ingredient volume of the dose order, subject to various exceptions, described in greater detail below.

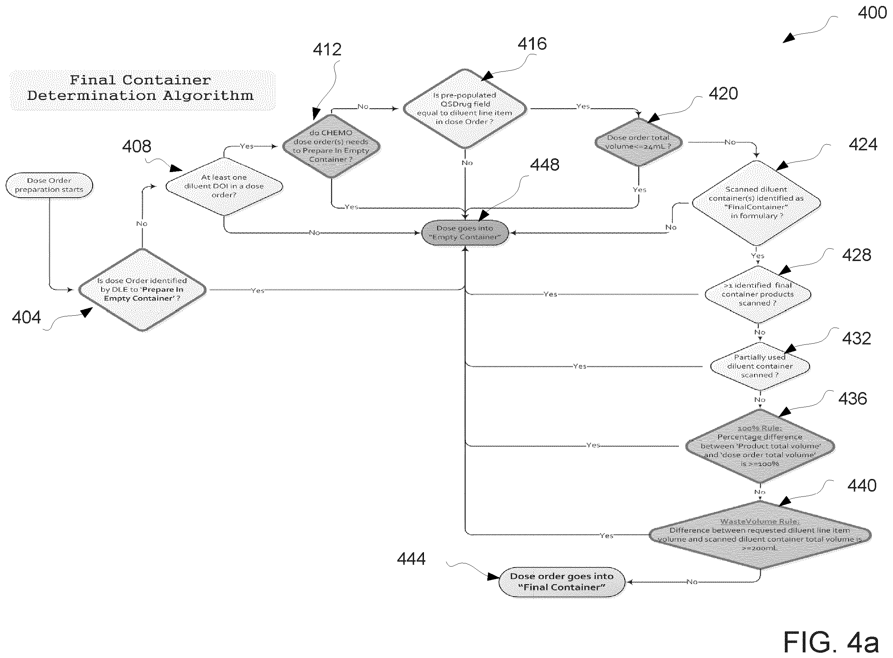

Turning now to Figs. 4a-4e, the pharmacy workflow application 114 may dynamically determine a final container of the dose order based at least in part on a final container characteristic in relation to a plurality of logical steps according generally to the logical steps 400. Each of the plurality of logical steps may reference at least one data value or attribute of the final container characteristic in order to carry out the logical step. In some regards, the plurality of logical steps may be viewed as a checklist of requirements such that each requirement must be satisfied in order for the selected product of the dose order to constitute a final container. The failure of any one of the plurality of logical steps may result in the dose order being prepared in a new or empty container. The dose order may be prepared in the new or empty container even in the case where some of the logical steps are satisfied - *i.e.,* all logical steps must be satisfied in order for the selected container to constitute a final container. The logical steps may be performed in the illustrated sequences or in other sequences accordingly.

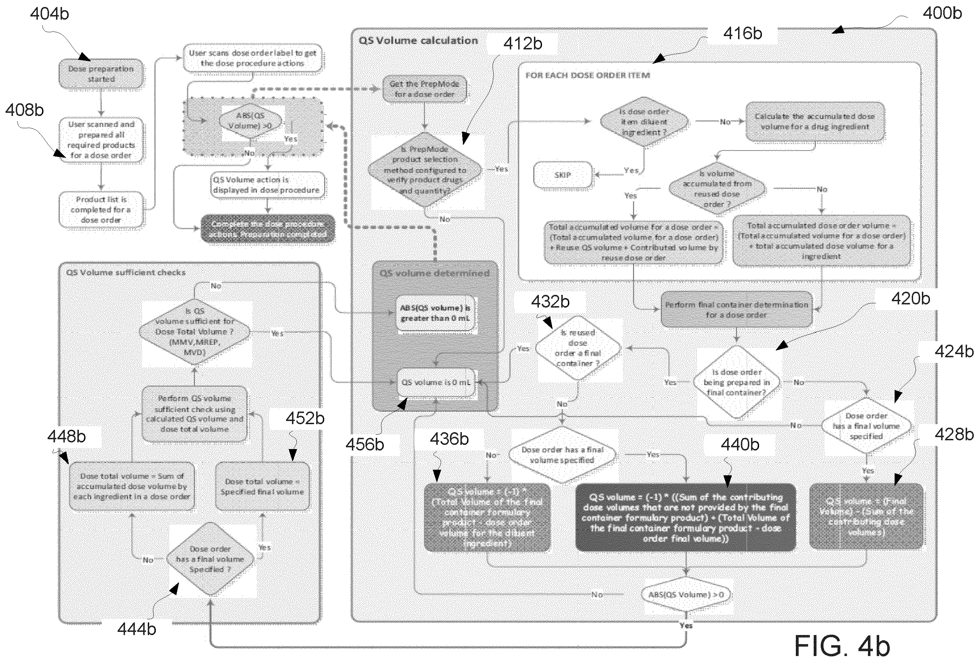

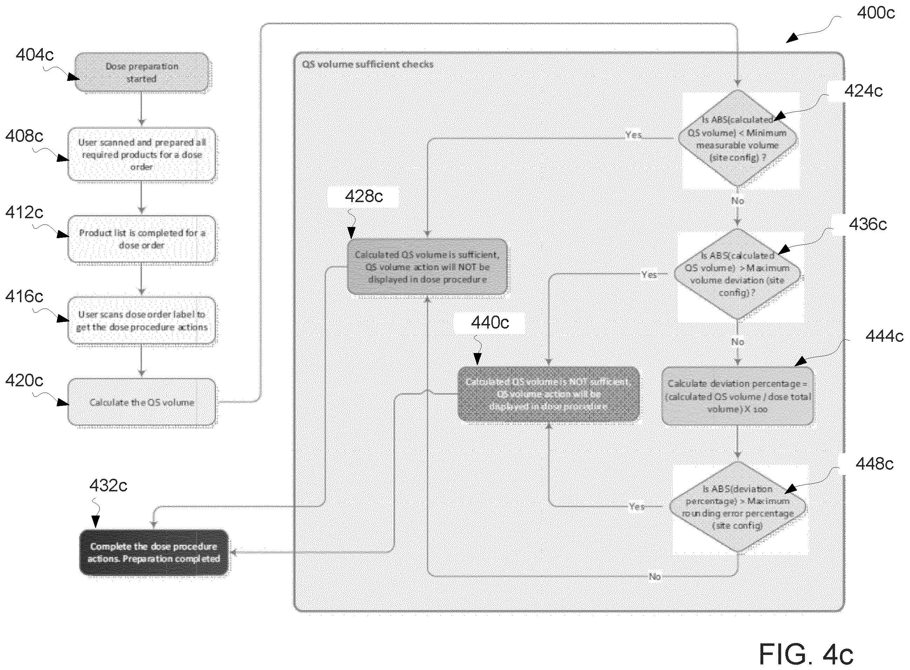

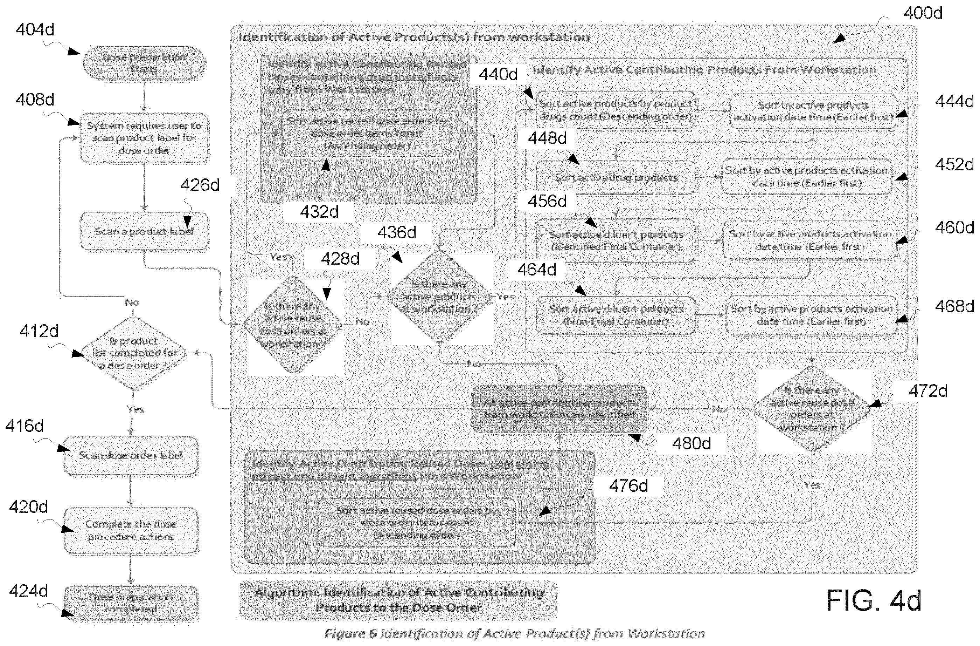

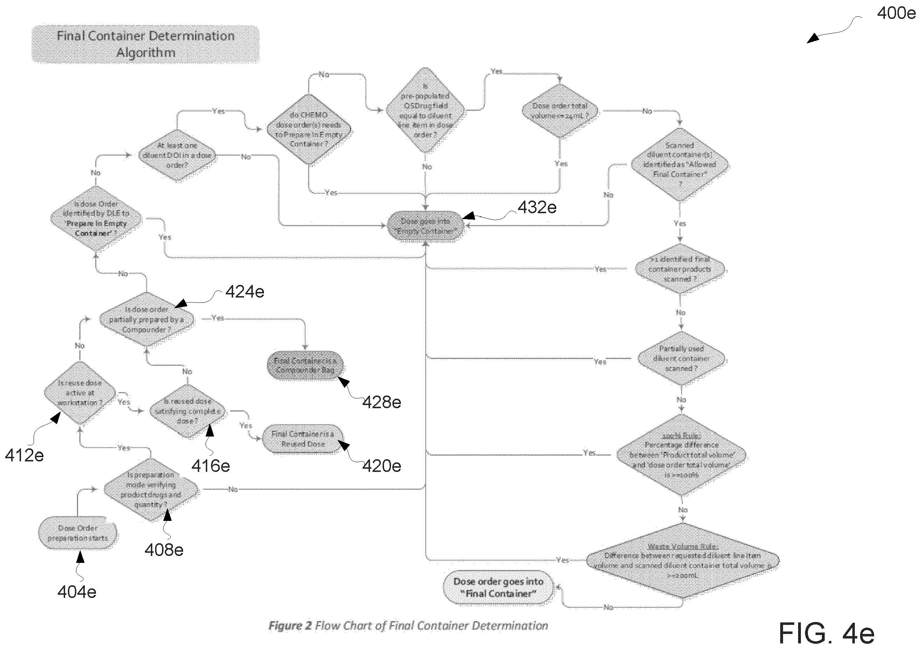

In one embodiment, the plurality of logical steps may comprise the logical steps 400 illustrated in Fig. 4. The logical steps may proceed such that a subsequent logical step may be executed upon the satisfaction of a previous logical step. It will be appreciated that the logical steps may include other logical steps, or omit one or more of the following described logical steps 400, as needed to dynamically determine the final container based on said final container characteristic.

The logical steps 400 may reference various data attributes in dynamically determining the final container. In some instances, the data attribute may include a data field corresponding to a dose order record. In other instances, the data attribute may include a data field corresponding to a formulary record associated with at least one of the plurality of indicated products. In this regard, the formulary record associated with at least one of the plurality of indicated products may include a data field at least partially for the purpose of the pharmacy workflow application 114 dynamically determining the selected product as the final container. As a further example, each formulary product may be marked as a drug, diluent, or both a drug and diluent *(e.g.,* a pre-mix product). Additionally, chemotherapy or hazmat products may be flagged or indicated using a specific indication in the formulary record data field. Further, the formulary data record may indicate that only a product marked or indicated as a diluent or a drug/diluent can be used as a final container. As such, these products may be specifically indicated as a "final container" in the formulary to indicate that the product is eligible for us as such. Also, a product with a product volume less than or equal to a selectable, predetermined volume *(e.g.,* 5, 10, 20, or 25 ml or less, etc.) may be indicated as not eligible as a final container or a "non-final" container. Further still, products that are clinically deemed as not suitable to be final containers may be indicated as a non-final container. In some embodiments, other data fields may be included in the formulary data record for purposes of making the final container determination. Moreover, the specific values may be configurable for site-specific preferences. In any event, these data fields of the formulary data record may be accessed by one or more of the logical steps 400 to facilitate the pharmacy workflow application 114 dynamically determining the final container.

In this regard, pharmacy workflow application 114 may execute a logical step 404 to determine whether the dose order is associated with a data attribute corresponding to an indicator indicative of the suitability of the dose order to be prepared in a final container. In some instances, logical step 404 may be referred to as a "Dose Logic Engine" rule or DLE rule. If the dose order does not include the indicator indicative of the suitability of the dose order to be prepared in a final container (e.g., expressly or by reference to a specific ingredient name in the dose order), the dose order will be prepared a new or empty container 448. That is, absent a positive indication of the suitability of the dose order for preparation in a final container, the selected dose order may not be prepared in a final container. This may be the case, for example, where the dose order is specifically indicated for preparation in a new or empty container. Upon the positive identification of the final container indication of the dose order, the pharmacy workflow application 114 may proceed to logical step 408. Otherwise, the pharmacy workflow application 114 may proceed to logical step 448 such that the dose order is prepared in the empty container.

In this regard, pharmacy workflow application 114 may execute a logical step 408 to determine whether the dose order is to include at least one diluent. That is, for a dose order record to be eligible to undergo the dynamic final container determination, at least one of the plurality products associated with preparation may correspond to a diluent product. Accordingly, it may be that only a product container including a diluent may be selected as a final container. That is, absent a diluent container being required for preparation of the dose order, the selected product container may not be used as a final container. As such, diluent-free dose orders may be prepared in a empty or new container 448. Upon the positive identification of a diluent in the dose order, however, the pharmacy workflow application 114 may proceed to logical step 412. Otherwise, the pharmacy workflow application 114 may proceed to logical step 448 such that the dose order is prepared in the empty container.

In this regard, pharmacy workflow application 114 may execute a logical step 412 to determine whether the dose order is associated with a formulary product having a data attribute corresponding to an indicator indicative of a "hazmat" drug. This may be the case, for example, where the dose order includes a drug intended for use in a chemotherapy treatment. Accordingly, a dose order associated with a hazmat drug may not be suitable for preparation in a final container. Rather, a dose order including a hazmat drug may be prepared in a new or empty container 448. In some embodiments, logical rule 412 may be configurable based on site-specific preferences. For example, the hazmat drug classification may be modified to include specific types of hazmat drugs or to distinguish between one category of hazmat drugs which may be prepared in a final container, and another category of hazmat drugs which may not be prepared in a final container. Furthermore, each product in a formulary may comprise an indication of either a hazmat drug or not a hazmat drug. Upon the positive identification of the suitability of the dose to undergo the final container determination based on a hazmat drug indicator, the pharmacy workflow application 114 may proceed to logical step 416. Otherwise, the pharmacy workflow application 114 may proceed to logical step 448 such that the dose order is prepared in the empty container.

In this regard, pharmacy workflow application 114 may execute a logical step 416 to determine whether the dose order is associated with a pre-populated "QS Drug" field equal to a diluent line item in the dose order. In this regard, the QS Drug field must equal the diluent line item in the dose order in order for the selected product to constitute a suitable final container. 448. That is, if the dose order includes a drug that requires a different carrier *(i.e.,* a QS Drug), than the diluent ingredient in the dose order to fulfill the dose, the dose may be prepared in to an empty container. As such logical step 416 verifies that the product contains the request volume of product to satisfy the requirements of the dose order. Absent this equality, the dose order will be prepared in an empty or new container, absent any addition adjustments to the product (e.g., volume addition or withdraw from the product). For example, a hypothetical preparation of Cefazolin 1000 mg, NS 250 ml in 250 ml, after scanning all products, may include a calculated QS volume of -10 ml *(i.e.,* inverse QS). In order to satisfy the dose order total volume *(i.e.,* final volume) 250 ml request, the pharmacy workflow application 114 will display a preparation step that instructs a user to remove 10 ml (QS volume) from the final container product. As such, the remove of the 10 ml may permit the 250 ml product to be used as a final container in the foregoing dose order example. Upon the positive identification of the noted equality, the pharmacy workflow application 114 may proceed to logical step 420. Otherwise, the pharmacy workflow application 114 may proceed to logical step 448 such that the dose order is prepared in the empty container.

The pharmacy workflow application 114 may execute a logical step 420 to determine whether the dose order comprises a total volume equal to or less than a minimum dose volume *(e.g.,* less than 5 ml, 10 ml, 24 ml, 30 ml, 35 ml etc.). If the total volume of the dose order is less than or equal to the minimum dose volume the dose may be prepared in an empty or new container 448. Accordingly, logical rule 420 may be configurable based on site-specific preferences. For example, the minimum dose volume may be selectable prior to dose preparation of a given dose. Upon the positive identification of the volume of the dose order exceeding the minimum dose volume, the pharmacy workflow application 114 may proceed to logical step 424. Otherwise, the pharmacy workflow application 114 may proceed to logical step 448 such that the dose order is prepared in the empty container.

In this regard, pharmacy workflow application 114 may execute a logical step 424 to determine whether the dose order includes a requirement for a diluent product associated with a formulary record data attribute corresponding to an indicator that the diluent product is suitable as a final container. If the diluent product of the dose order is not indicated as being suitable as a final container (e.g., as recorded in a formulary record corresponding to one diluent product), the dose order will be prepared a new or empty container 448. This may be the case, for example, where the diluent formulary product is in a stored in a vial and is indicated in a corresponding formulary record as not being suitable as a final container. Upon the positive identification of the diluent final container indication, the pharmacy workflow application 114 may proceed to logical step 428. Otherwise, the pharmacy workflow application 114 may proceed to logical step 448 such that the dose order is prepared in the empty container.

In this regard, pharmacy workflow application 114 may execute a logical step 428 to determine whether the dose order includes more than one indentified final container products. That is, if the dose order contains multiple products as indicated as being diluent products, any one of which could theoretically constitute a final container, the dose order will be prepared in a new or empty container 448. That may be due, in part, because only one diluent product may constituent the final container. Upon the positive identification of the existence of only one indicated diluent product which may constitute a dynamically determined final container, the pharmacy workflow application 114 may proceed to logical step 432. Otherwise, the pharmacy workflow application 114 may proceed to logical step 448 such that the dose order is prepared in the empty container.

In this regard, pharmacy workflow application 114 may execute a logical step 432 to determine whether the selected product corresponding to the potential final container is not a partially used diluent container. Accordingly, only a new or unused diluent product may constituent a final container. As such, where the indicated diluent formulary product corresponds to a partially used diluent formulary product *(e.g.,* a diluent formulary product used, in part, to prepare a prior dose order or a "work-in-progress" ("WIP") product), the dose order may be prepared in a new or empty container 448. Upon the positive identification that the selected product corresponds to a new or unused diluent formulary product, the pharmacy workflow application 114 may proceed to logical step 436. Otherwise, the pharmacy workflow application 114 may proceed to logical step 448 such that the dose order is prepared in the empty container.

In this regard, pharmacy workflow application 114 may execute a logical step 436 to determine whether the dose order satisfies a volume differential rule for a product being evaluated as a final container. That is, in order to satisfy the volume differential rule, the absolute difference between the diluent formulary product volume in its original container and the dose order total volume may not exceed a specified and predetermined differential percentage. The calculation of the differential percentage may be represented according to the following expression: [(diluent product total volume - dose order total volume)/(dose order total volume)] * 100. If the foregoing expression returns a value greater than or equal to the specified differential percentage, the dose order may be prepared in a new or empty container 448. Preparing the dose order in a new or empty container may also enable using the remaining volume of the indicated product for future doses through assigning the product a work-in-process (WIP) label and designation in the formulary along with the appropriate expiration date as defined in the product formulary. In some embodiments, logical rule 436 may be configurable based on site-specific preferences. For example, the specified differential percentage may be selectable *(e.g.,* 50%, 75%, 100%, 125%, etc.). Upon the positive identification that the expression returns a value less than the specified differential percentage, the pharmacy workflow application 114 may proceed to logical step 440. Otherwise, the pharmacy workflow application 114 may proceed to logical step 448 such that the dose order is prepared in the empty container.

In this regard, pharmacy workflow application 114 may execute a logical step 440 to determine whether the dose order satisfies the waste volume rule. That is, in order to satisfy the waste volume rule, the absolute difference between the dose order carrier ingredient volume and the diluent product total volume may not exceed a selectable and predetermined waste volume. That calculation of the waste volume may be represented according to the following expression: (dose order carrier ingredient volume - diluent product total volume). If the foregoing expression returns a value greater than or equal to the waste volume, the dose order may be prepared in a new or empty container 448. Preparing the dose order in a new or empty container may also enable using the remaining volume of the product for future doses through assigning the product a work-in-process (WIP) label along with the appropriate expiration date as defined in the formulary data record. In some embodiments, logical rule 440 may be configurable based on site-specific preferences. For example, the waste volume may be selectable (e.g., 100 ml, 200 ml, 300 ml, etc.). For illustration, upon the positive identification that the expression returns a value less than 200 ml, the pharmacy workflow application 114 may determine that the selected product of the dose order may constitute the final container 444. Otherwise, the pharmacy workflow application 114 may proceed to logical step 448 such that the dose order is prepared in the empty container.

It will be appreciated that any or all of the foregoing logical rules may be applied in a multitude of use cases. That is, the logical rules may dynamically determine the final container in various circumstances based on the particular requirements of the initiated dose preparation protocol and to the received indication of the plurality of products to be used to prepare the dose. In this regard, various use cases are presented for the sake of illustration below. The presented use cases are merely examples and should not be read to limit the applicability of the logical rules 400 to any particular use case or dose preparation protocol.

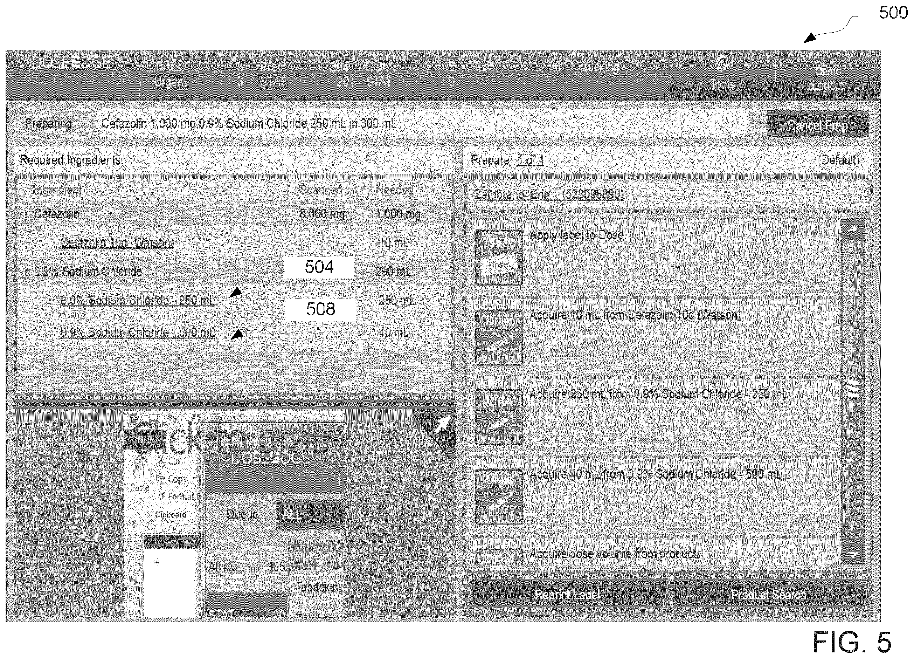

In this regard, with reference to Fig. 5, a user interface 500 illustrating an exemplary use case may comprise dynamically determining a final container in a dose preparation protocol that includes a scenario wherein multiple diluent product formulary products suitable as a final container are indicated or scanned as part of a dose preparation protocol. For instance, the 0.9% Sodium Chloride 250 ml diluent product 504 and the 0.9% Sodium Chloride 500 ml diluent product 508 may both be utilized as a final container. In this instance, and with reference to the logical rule 428, neither of the scanned diluent formulary products may constituent the final container of the dose order. Rather, the dose order will be prepared in a new or empty container.

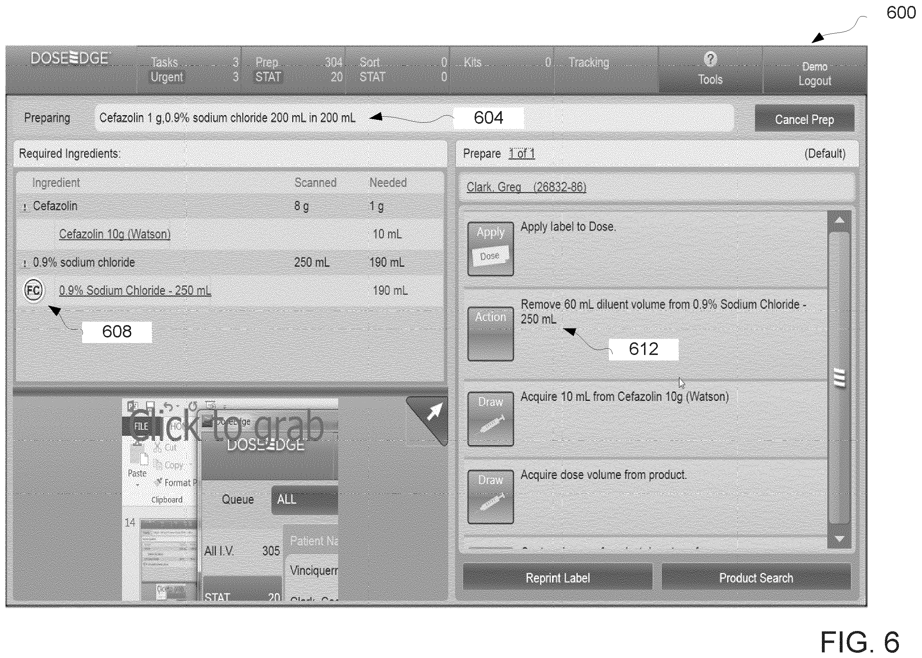

In this regard, with reference to Fig. 6, a user interface 600 illustrating another use case may comprise dynamically determining a final container in a dose preparation protocol that includes a negative QS volume with a final specified volume. A QS volume may reference the volume of diluent need to reconstitute the dose as described above in relation to Fig. 4b. With continued reference to the example in Fig. 6, a Cefazolin 1 g, 0.9% Sodium Chloride 200 ml in 200 ml dose order is specified 604. In this instance, a 250 ml bag of 0.9 % Sodium Chloride is scanned and dynamically determined to constitute the final container for the dose order 608. In this instance, the pharmacy workflow application 114 may calculate 1 negative QS volume of 60 ml based on the contributed diluent for the Cefazolin. With reference to logical rule 440, the waste volume of the dose order *(i.e.,* 60 ml) does not exceed a predetermined waste volume, in this case, 250 ml. As such, 60 ml of diluent is withdrawn from the foregoing 250 ml bag 612, such that the bag will constituent the final container after the addition of the Cefazolin drug.

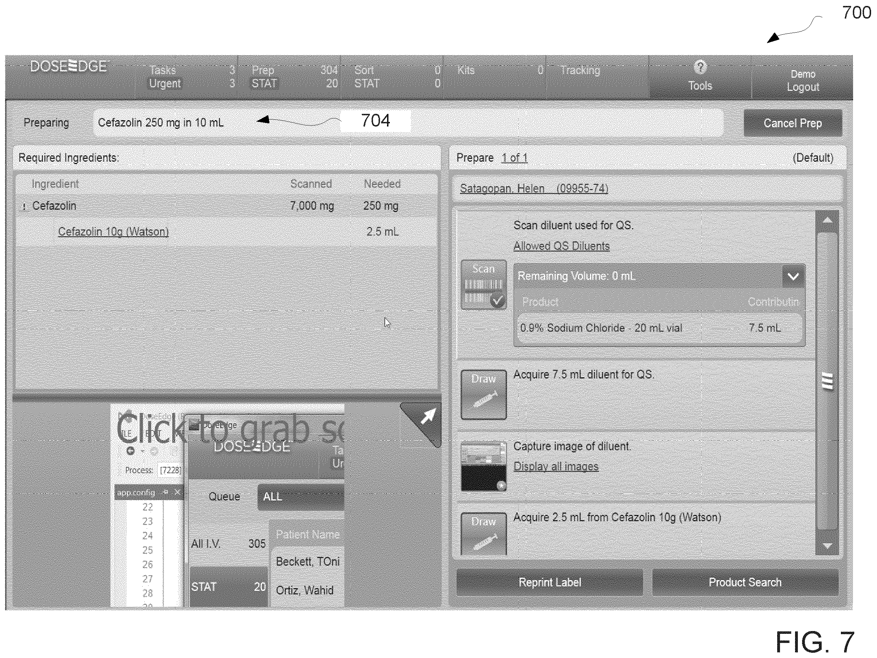

In this regard, with reference to Fig. 7, a user interface 700 illustrating still another use case may comprise dynamically determining a final container in a dose preparation protocol that includes no diluent line item in the order. For example, and with continued reference to Fig. 7, the dose order may include a request for: "Cefazolin 250 mg in 10 ml," 704. In this regard, and with reference to logical rule 408, because the dose order does not contain in indication corresponding to a diluent product, the dose order may be prepared in a new or empty container.

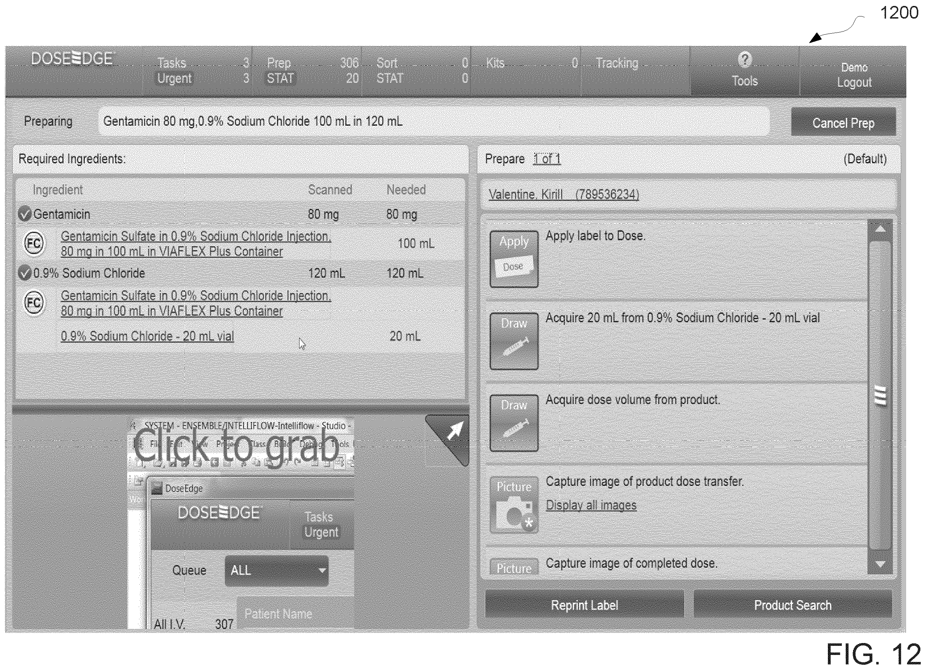

In this regard, another case may comprise dynamically determining a final container in a dose preparation protocol that includes a premix product. A premix product may include a formulary product premixed with a drug and diluent to a specified concentration. In this regard, a dose order may, in some cases, comprise only a request for a premix product, and thus, the premix product need not be altered as part of a dose preparation protocol. Where the premix product is not altered *(i.e.,* the premix satisfied the dose order entirely), the premix product itself is the final container. In other instances, a dose order may comprise a request for medication which may be prepared based at least in part on a premix product. Consider, with reference to Fig. 12, a user interface 1200 according to another use case, which displays a hypothetical dose order 1204 comprised of: Gentamicim 80 mg, 0.9 % Sodium Chloride 100 ml in 120 ml. In this regard, the pharmacy workflow application 114 may receive an indication corresponding to premix product 1208 comprised of a Gentamicin Sulfate in 0.9% Sodium Chloride Injection, 80 ml in 100 ml in VIAFLEX Plus Container. In this regard, the premix product may be identified as the final container 1212 as noted by the encircled "FC." In this particular example, the premix product must be QS'd (*e.g.,* supplied with additional diluent) in order to reach the final volume specified of 120 ml, as depicted at 1216, showing 0.9% Sodium Chloride 20ml added to the premix product.

Turning next to Fig., 4b, the pharmacy workflow application 114 may adjusting the diluent ingredient volume associated with the dose preparation protocol according generally to method 400b. In this regard, the method 400b may proceed generally in the following manner. Pharmacy workflow application 114 receives 404b a dose order awaiting preparation and initiates the dose order preparation protocol. The pharmacy workflow application may receive an indication, in the form of a scan 408b of the dose order label corresponding to the required ingredients. For example, the received indication may correspond to a pharmacy technician taking possession of the product for use the preparation of the dose. Next, the preparation protocol is checked 412b for having a product selection method configured to verify product drugs and quantity. Upon a positive indication of such, the pharmacy workflow application calculates 416b the total accumulated dose order volume of non-diluent ingredients. Next, the pharmacy workflow application 114 verifies 420b that the dose order is set for preparation in a final container. The system may then ascertain 424b whether the dose order is associated with a final specified volume. Notably, if the dose order does not both have final container product scanned and final volume of the dose order specified, system will not request to determine a QS volume for the dose order 456b. If, however, the dose order does not have final container scanned, but the final volume of dose order is specified, the pharmacy workflow application 114 may QS 428b the dose order such that the QS volume is determined by reference to following expression: (Final Volume) - (Sum of the contributing dose volumes).

In some embodiments *(i.e.,* where the dose order is identified as being prepared in a final container), the pharmacy will ascertain 432b whether a reused dose order is selected for a final container. If so, the pharmacy workflow application 114 may not determine a QS volume for the dose order 456b. In other embodiments, the reused dose order may not be a final container for the dose order. In this regard, the dose order is checked to see whether the dose order is associated with a final specified volume. As such, and according to step 436b, if reused dose order is not a final container for dose order, then the dose order is checked for having the final volume specified. If the final volume is not specified, system will request 436b to determine a QS volume for the dose order such that the QS volume is determined by reference to following expression: [(-1) * (total volume of the final container formulary product - dose order volume for the diluent ingredient)]. Conversely, if the reused dose order is not a final container for dose order and the final volume is specified, the pharmacy workflow application 114 may determine 440b a QS volume for the dose order such that the QS volume is determined by reference to following expression: (-1)*[(sum of the contributing dose volumes that are not provided by the final container formulary product) + (total volume of the final container formulary product - dose order final volume)].

As a result of the pharmacy workflow application 114 determining that the QS volume is greater than zero, the pharmacy workflow application may perform one or more QS volume sufficiency checks. Specifically, this may begin by pharmacy workflow application 114 determining 444b whether the dose order has a final volume specified. To the extent that the dose order does not have a final volume specified 448b, the dose order total volume may be determined by reference to the following equation: dose total volume = sum of accumulated volume by each dose order ingredients. However, if the dose order does have a final volume specified 452b, the dose total volume is determined by reference to the following equation: dose total volume = specified dose order final volume.

Turning next to Fig. 4c, the pharmacy workflow application 114 may determine if the QS volume will be displayed in part by performing a QS volume sufficient check according generally to method 400c. Method 400 may proceed by initiating the dose preparation at step 404c. This may correspond to the pharmacy workflow application 114 initiating the dose preparation protocol corresponding to the dose order. Next, the pharmacy workflow application 114 may receive an indication or a scan and prepare all required products for the dose order according to step 408c. The method 400c may continue at step 412c such that the product list is completed for a dose order. The method 400c may continue at step 416c such that the user scans a dose order label to get the dose order procedure actions. The method 400c may continue at step 420c such that the QS volume is calculated *(e.g.,* by method 400b, as depicted in Fig. 4b). The method 400c may continue at step 424c such that the absolute value of the calculated QS volume is compared to the minimum measureable volume (e.g., according to the site configuration parameters). To the extent that the absolute volume of the calculated QS volume is less than the minimum measureable volume, the method 400c may continue at step 428c such that it is determined that the calculated QS volume is sufficient. In this regard the QS volume action will not be displayed in the dose procedure and the method 400c may conclude at step 432c such the preparation of the dose procedure is complete.

However, with returned reference to step 424c, to the extent that the absolute value of the calculated QS volume is greater than the minimum measurable volume, the method 400c may continue at step 436c such that the absolute value volume is compared to the maximum volume deviation (e.g., according to the site configuration parameters). To the extent that the absolute value of the calculated QS volume is greater than the maximum volume deviation, the method 400c may continue at step 440c such that the pharmacy workflow application 114 determines that the calculated QS volume is not sufficient. In this regard, the dose preparation actions are displayed at step 432c. However, to the extent that the absolute value of the calculated QS volume is less than the maximum volume deviation, the method 400c may continue at step 444c such that the pharmacy workflow application 114 calculates the deviation percentage according to the following expression: (calculated QS volume / dose total volume) *100. The method 400c may continue at step 448c such the absolute value of the calculated QS volume is compared to a maximum rounding error percentage (e.g., according to site configuration parameters). To the extent that the absolute value of the calculated QS volume is greater than the maximum rounding error percentage, the method may continue at step 440 such the pharmacy workflow application 114 determines that the calculated QS volume is not sufficient. In this regard, the dose preparation actions are completed at step 432c. If, however, the absolute value of the calculated QS volume is less than the maximum rounding error percentage, the method 400c may continue at step 428c, such that the pharmacy workflow application 114 determines that the QS volume is sufficient such that the QS volume will not be displayed in the dose procedure. In this regard, the method 400c concludes at step 432.

Turning next to Fig. 4d, as previously noted, the pharmacy workflow application 114 may identify the currently active workstation products for the dose order in the particular dose order as part of the received indication according generally to method 400d. The method 400d may begin at step 404d such that the pharmacy workflow application 114 initiates the dose preparation protocol corresponding to the dose order. The method may continue at step 408d such that the system requires the user to scan a product label associated with the dose order. As such, step 408d may be performed relative to the foregoing received indication of a plurality of products associated with the dose preparation protocol. The method 400d may continue at step 426d such the labels of the plurality of products are scanned. The method 400d may continue by the pharmacy workflow application 114 determining if there are any active reuse dose orders at the workstation. If so, the method 400d may continue at step 432d by identifying active contributing reused doses containing drug ingredients from the workstation. This may be accomplished by sorting the active reused dose orders by dose order item count in ascending order. Regardless of any active reuse dose orders are present at the workstation, the method 400d may continue at step 436 by pharmacy workflow application 114 determining whether there are any active products at the workstation. To the extent that there are active products at the workstation, the method 400d may identify active contributing products from the workstation.

Specifically, the pharmacy workflow application 114 may identify active contributing products from the workstation by executing step 440d of method 400d such that the active products are sorted by product drug count in descending order. The method 400d may continue at step 444d such that the active products are sorted by activation date time with the earliest time being ranked first. The method 400d may continue at step 448d such the active drug products are sorted. The method 400d may continue at step 452d such that active products are sorted by activation date time with the earlier time being ranked first. The method 400d may continue at step 456d such the active diluent products are sort according to the identified final container indication. The method 400d may continue at step 460 such that the active products are sorted by activation date time with the earlier time being ranked first. The method 400d may continue at step 464d such that the active diluent products are sorted according to the identified non-final container indication. The method 400d may continue at step 468d such the active products are sorted by activation date time with the earlier time being ranked first. The method 400d may continue at step 472d, wherein the pharmacy workflow application 114 may determine if there are any active reuse dose orders at the workstation. To the extent that active reuse dose orders are at the workstation, the pharmacy workflow application 114 may identify active contributing reused dose order containing at least one diluent ingredient from the workstation. This may be accomplished by sorting the active reused dose orders by dose order items count in ascending order.

The method 400d may continue at step 480d such that all active contributing products from the workstation are identified. With returned reference to step 436d, to the extent that there are not any active products at the workstation, the method 400d may also proceed to step 480d. In either event, the method 400d may proceed to step 412d, such the pharmacy workflow application 114 determines if the product list is completed for the dose order. To the extent that the product list is not completed for the dose order, the method 400d may continue at previously discussed step 408d. However, to the extent that the product list is complete for the dose order, the method 400d may continue at step 416d such that the label of the dose order is scanned. The method 400d may continue at step 420d such that the dose procedure actions are completed. The method may continue at step 424d such that the dose preparation is completed.

Turning next to Fig. 4e, in other embodiments, the pharmacy workflow application 114 may execute a series of steps, according generally to method 400e, associated with an embodiment of the dynamically determined final container determination f the logical steps 400 depicted in Fig. 4. In this regard, method 400e may begin by initiating the dose order preparation. In some instances, this may correspond to initiating a dose preparation protocol corresponding to the dose order. The method 400e may continue at step 408e such that pharmacy workflow application 114 determines if the dose order will be prepared via a preparation mode that will verify the product drugs and quantity. To the extent that the dose order will not be prepared via a preparation mode that will verify the product drugs and quantity, the dose order may be prepared in an empty or new container 432e. However, to the extent that the dose order will be prepared via a preparation mode that will verify the product drugs and quantity, the method 400e may continue at step 412e such that the pharmacy workflow application 114 may determine if a reuse dose is active at the workstation. To the extent that the reuse dose is active at the workstation, the method 400e may continue to step 416e such that the pharmacy workflow application determines if the reused dose satisfies a complete dose. To the extent that the reused dose satisfies a complete dose the method 400e may continue at step 420e such that the final container is determined to be the container of the reused dose. If, however, and with returned reference to steps 412e and 416e, the reused dose does not satisfy a completed dose or there is no reuse dose active at the workstation, the method 400e may proceed to step 424e such that the pharmacy workflow application 424e may determine if the dose order is partially prepared by a compounder. To the extent that the dose order is partially prepared by the compounder, the method 400e may continue at step 428e such the final container is determined to be the compounder bag. If, however, the dose order is not partially prepared in a compounder the method 400e may continue according to the method 400.

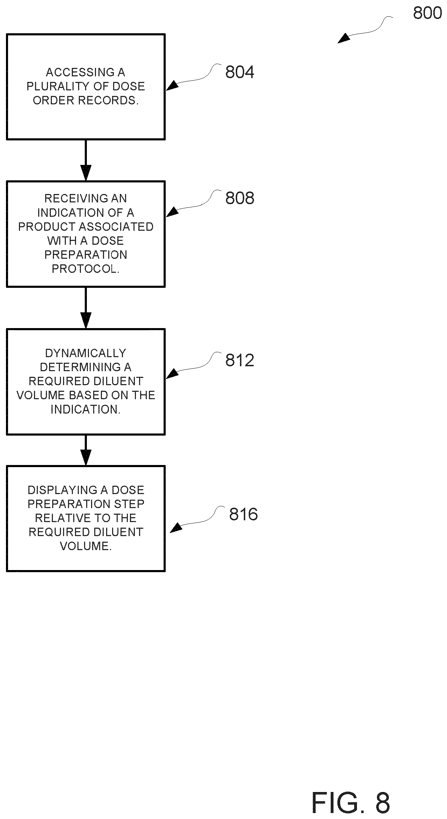

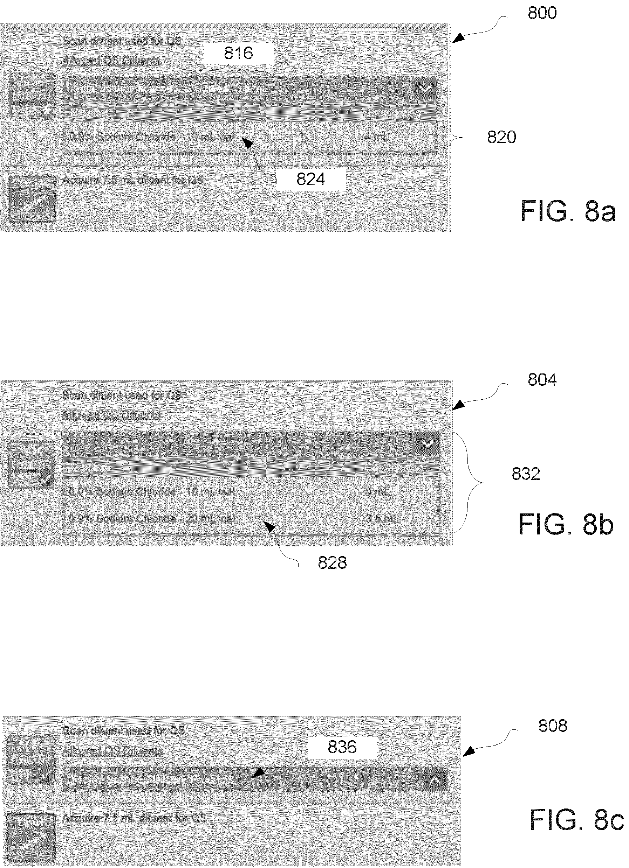

Turning next to Fig. 8, according to another embodiment, the pharmacy workflow application 114 may facilitate dynamic diluent volume accounting in a dose preparation protocol according generally to the method 800. In this regard, the diluent volume may be dynamically adjusted based on the receipt of an indication of a product associated with a dose preparation protocol. Accordingly, the pharmacy workflow application may display to a user a precise amount of required diluent volume for a given dose order selected for preparation. And in some embodiments, the required diluent volume may be fulfilled by multiple separate diluent products such that the additive volume amount of the multiple separate diluent product containers is at least equal to the dynamically determined required diluent volume.

In some instances, the preparation of dose orders with dynamic diluent volume accounting may facilitate efficient dose order preparation techniques and contribute to overall error reduction. In one embodiment, the dynamic diluent volume account allows the pharmacy workflow application 114 to receive an indication (e.g., a scan) associated with multiple bag of the same diluent ingredient to prepare the required diluent volume for the dose preparation protocol. The received indication may establish the presence, for example, of the diluent products required to prepare the dose at a preparation workstation. In this regard, a dose order may be prepared from multiple sources of diluent. This aspect may increase efficiency by facilitating a workflow that allows a user to prepare a dose order with various volumetric combinations of identical diluent products. Moreover, other aspects of the dynamic diluent volume accounting may reduce dose preparation errors. For example, according to another embodiment, the pharmacy workflow application 114, via the user interface 108, may display a running total of the volume of the diluent product required according to one or more preparation steps, which demonstrates the physical steps required to complete the dose preparation. In this regard, errors associated with using the wrong volume of diluent during dose preparation may be reduced.

The dynamic alteration of the volume includes the pharmacy workflow application 114 accessing 804 a dose order record corresponding to a dose order stored in, for example, local database 112. The dose order record may correspond to a dose order received by the pharmacy workflow application 114 that has not yet been prepared. Furthermore, the dose order may include a request for medication that is awaiting preparation in the pharmacy workflow application 114. In some instances, the corresponding dose order record of the dose order may be stored in a queue comprised of a plurality of dose order records with corresponding dose orders also awaiting preparation. In either event, in this embodiment, the pharmacy workflow application accessed a single dose record.

The pharmacy workflow application 114 may receive 808 an indication of a product associated with a dose preparation protocol. The dose preparation protocol may include, among other things, a list of ingredients or a "recipe" which communicates the constituent components of a dose order. In this embodiment, the preparation protocol may include at least one drug product and at least one diluent product. The at least one drug product may correspond to a pharmaceutical drug or active medication ingredient for therapeutic effect. The at least one diluent product may correspond to a reconstitution solution for use in diluting the concentration of the product. In this regard, a dose order may specify a precise amount of product *(i.e.,* medication to be delivered) volume, and require the diluent to be added to the product in order to reach a final volume of the dose order, for instance. The diluent added in this regard, may constitute a QS volume, or a volume of diluent (in ml) added to a product to complete the dose order. The QS volume, as disused in more detail below, may change depending on a variety of factors, including the initial product concentration, requested final volume, and the like.

The received indication of the product associated with the dose preparation protocol may facilitate the pharmacy workflow application 114 dynamically determining 812 a required diluent volume based on the received indication. That is, the indication may include data attributed of the drug corresponding to the drug product. The data attributes may include, among other things, the concentration and/or volume of the formulary drug product. Moreover, this indication may be received by the pharmacy workflow application 114 in the context of the dose preparation protocol and associated dose order. In any event, the foregoing defined QS volume may be dynamically determined based on the data attributes of the indication.

It will be appreciated that the dynamic determination of the QS volume may be application in a variety of cases based on various data attributes. For the sake of illustration, the following discussion highlights the pharmacy workflow application 114 dynamic alteration of the QS volume based on two specific data attributes: final volume of the dose order, and initial volume of the formulary drug product. Notably, the dynamic determination is not limited to either of the following two data attributes.

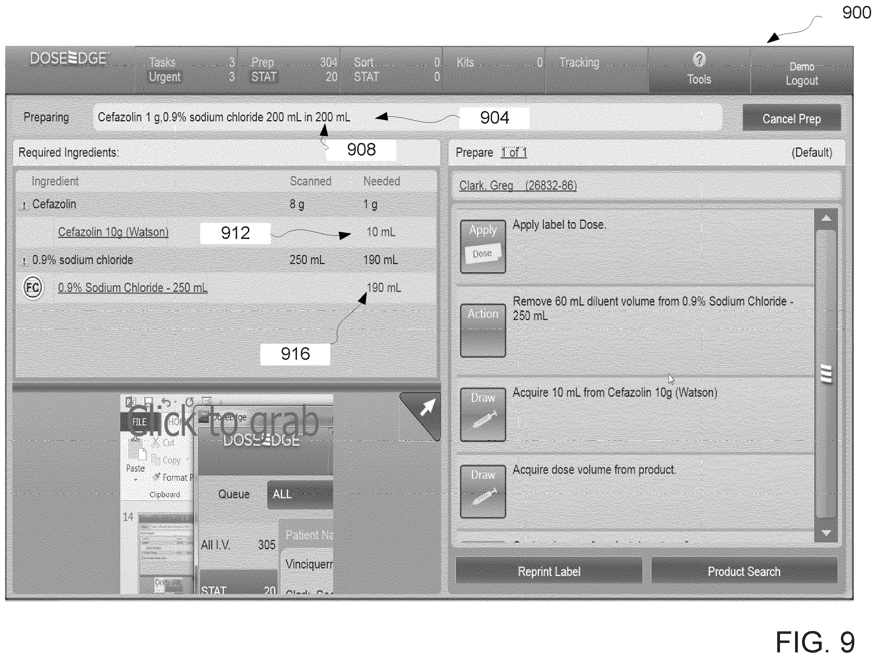

In this regard, with brief reference to Fig. 9, the QS volume may be dynamically determined based on the final volume specified in the dose order as generally indicated by user interface 900. For example, a dose order may include a dose 904 of: Cefazolin 1 g, 0.9% sodium Chloride 200 ml in 200 ml. As such, the dose order of the example specified a final volume 908 of 200 ml. The received indication corresponding to a formulary record corresponding to the Cefazolin drug product notes a Cefazolin dose at a concentration of 100 mg/ml (not pictured). The dynamic determination will use this information to determine that the Cefazolin drug will constituent 10 ml of the 200 ml final volume (*i.e.,* 1000 mg / (100 mg/ml) = 10 ml) 912. As such, the pharmacy workflow application 114 may determine that the required diluent volume 916 for this particular dose order equate 190 ml *(i.e.,* 200 ml - 10 ml = 190 ml). For the sake of continued illustration, consider that the required diluent volume would change in the event that the final volumes specified in the dose order were different. That is, based on the logic of the foregoing determination, a Cefazolin 1 g, 0.9% sodium Chloride 200 ml in 300 ml dose, would result in a dynamic determination of a required diluent volume of 290 ml, assuming the received indication of the formulary drug product is the same as that above (*i.e.,* 1000 mg / (100 mg/ml) = 10 ml; 300 ml - 10 ml = 290 ml).

In this regard, the QS volume may be dynamically determined based on the concentration of the indicated formulary record for a drug product received in connection with the dose preparation protocol of the dose order. For example, a dose order may include the dose of: Cefazolin 1 g, 0.9% sodium Chloride 200 ml in 200 ml. As such, the dose order of the example specified a final volume of 200 ml. The received indication corresponding to a formulary drug product may note a Cefazolin dose at a concentration of 50 mg/ml. The dynamic determination will use this information to determine that the Cefazolin drug will constituent 20 ml of the 200 ml final volume *(i.e.,* 1000 mg / (50 mg/ml) = 20 ml). As such, the pharmacy workflow application may determine that the required diluent volume for this particular dose order equate 180 ml (*i.e.,* 200 ml - 20 ml = 180 ml).

With return reference to Fig. 8, the previously noted interface 108 of local system 110 may also be operable to display 816 a dose preparation step associated with the preparation of the dose relative to the diluent volume. That is, the preparation step of the preparation protocol may be displayed at least partially based on the dynamically determined diluent volume. In this regard, the pharmacy workflow application 114 may communicate to a user the dynamically determined diluent volume such that the user may incorporate the dynamically determined diluent volume in the preparation of the dose order.

In some embodiments, the pharmacy workflow application 114 may receive one or more indications corresponding to the required diluent volume. In this regard, the pharmacy workflow application 114 may require the identification of the particular diluent product intended for use in the preparation of the dose order. Notably, in this embodiment, the required diluent volume may be satisfied by one or more diluent products. Each diluent product includes a diluent product volume. The pharmacy workflow application 114 may determine the diluent product volume of each diluent product in order to verify that the aggregate diluent formulary product volume is at least equal to the required diluent volume. In this regard, multiple diluent formulary products may be indicated in order to satisfy the dynamically determined required diluent volume.

The pharmacy workflow application 114 may, in the context of receiving an indication of the one or more diluent formulary products, display a running total of the volume of the diluent product yet needed to meet the required diluent volume. In this regard, the pharmacy workflow application 114 may communicate to the user the remaining volume of diluent formulary product required to satisfy the dynamically determined diluent volume. For example, a give dose order may require 300 ml of 0.9 % Sodium Chloride. Upon receipt of an indication of a diluent formulary product of 0.9% Sodium Chloride in a 250 ml bag, the pharmacy workflow application may indicate to the user that 50 ml are required to satisfy the required diluent volume *(i.e.,* 300 ml - 250 ml = 50 ml). The remaining volume indicator may appear on the user interface 108 associated with the pharmacy workflow application 114. The user interface 108 may also include a collapsible area for selectively displaying information associated with the one or more indications of the diluent formulary product. In this regard, the one or more diluent formulary products, appearing on the user interface as spate line items, may be collapsed into a single line that is indicative of the group of indicated diluent formulary products. Accordingly, the collapsible areas may facilitate organizing information such that the user interface 108 displays information to the user pertinent to the particular dose preparation protocol step of the dose order record (*e.g.,* the collapsible area may hide the various line items of the one or more diluent formulary products after the required diluent volume has been reached).

The foregoing indications may be displayed by the pharmacy workflow application 114 via user interface 108. User interface 108 may be configurable to present various indicators at least partially based on the received first indication of a product associated with a dose preparation protocol relative to the dynamic diluent volume accounting. With collective reference to Figs. 8a, 8b, and 8c, the configuration may resemble one or more configurations 800, 804, and 808. With specific regard to configuration 800 of Fig. 8a, user interface 108 is operable to display running total 816 indicative of the volume of the diluent product yet needed to meet the required diluent volume. The running total 816 may be calculated on the basis of one or more received indications of a product associated with a dose preparation protocol disposed on configuration 800 in region 820. For example, as illustrated in configuration 800, the dose preparation protocol required 7.5 ml of diluent Sodium Chloride (not pictured). Configuration 800, in this example, includes a first indication 824 of a product associated with a dose preparation protocol, in particular, corresponding to 0.9% Sodium Chloride in a 10 ml vial contributing 4 ml. In this regard, running total 816 indicates that 3.5 ml are yet required to meet the required diluent volume. As such, the pharmacy workflow application 114 may receive another indication 828, with reference to configuration 804 of Fig. 8b, to satisfied the running total amount. The user interface 108 may also include a collapsible area 832 for selectively displaying information associated with the indicated products. Notably, the information of the collapsible area may be temporality hidden from view upon selecting the collapsible area, as generally depicted by collapsed collapsible area 836 of configuration 812 of Fig. 8c. The collapsible area may facilitate dose order preparation by temporarily hiding information from the user when the information is not longer directly relevant to a particular step of the dose preparation protocol.

In another embodiment, the pharmacy workflow application 114 may determine whether an indicated diluent product is associated with a list of allowed diluent products relative to the dose preparation protocol. That is, various diluents may appropriately be used *(i.e.,* allowed diluents) in the preparation of a given dose preparation protocol (e.g., Sodium Chloride, etc.). The pharmacy workflow application 114 may display a dose preparation step at least partially based on a received indication of a diluent product associated with the list of allowed diluent products of the dose preparation protocol. As discussed in greater detail above, in some cases, the pharmacy workflow application 114 may receive multiple indications of diluent products intended to satisfy the required diluent volume. In this regard, upon the receipt of one indication of a diluent product, the required diluent volume must be satisfied by subsequent indications associated with the same diluent product. In this regard, after an indication of one diluent product, rather than determine if a subsequent diluent product is associated with a list of allowed diluent products of the dose preparation protocol, the pharmacy workflow application 114 may determine if the subsequent diluent product is the same diluent product as the first indicated diluent product. If any subsequently indicated diluent product is different than the first indicated diluent product, the subsequently indicated diluent product will not be able to satisfy the required diluent volume.

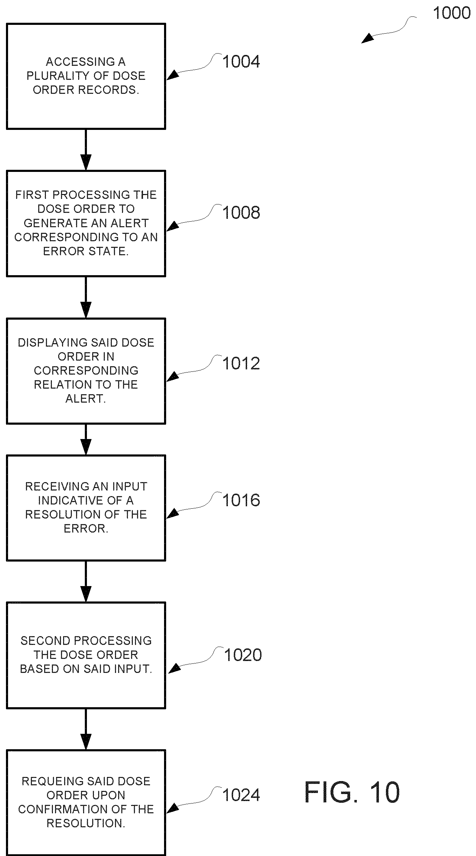

Turning next to Fig. 10, according to another embodiment, the pharmacy workflow application 114 may facilitate resolving critical issues associated with the pharmacy workflow management application 114 according generally to the method 1000. In this regard, the pharmacy workflow application 114 may be operable to identify various critical issues and display the identified critical issues in a common location to facilitate issue resolution. The identified critical issues may include various errors, such as an error regarding a dose order record, a password error, or other miscellaneous errors, as described in greater detail below. In this regard, according to one embodiment, errors associated with the dose order record may be identified and, in some instances, resolved within the pharmacy workflow queue. Accordingly, a dose order record with an error need not necessarily be prepared outside of the pharmacy workflow application.

In some instances, managing an error regarding a dose order in a pharmacy workflow queue may facilitate efficient dose order preparation techniques and contribute to error mitigation. Notably, in one embodiment, the pharmacy workflow application 114 may support user-resolution of errors within the pharmacy workflow application 114 framework. That is, an error associated with a dose order need not preclude the dose order from preparation via the pharmacy workflow application 114. Rather, the pharmacy workflow application 114 may identify the error and, in some cases, accept an input from an user indicative of a resolution of the identified error. In this regard, dose order efficiency may increase by facilitating the preparation of dose orders initially associated with errors. Furthermore, managing errors in this regard may also mitigate the occurrence of additional errors. This may occur by providing a combined critical issues display *(i.e.,* the Attention Tab, discussed in greater detail below) by which users may learn of various types of errors. Moreover, dose orders with identified errors may not be reintroduced in to the dose order queue without verifying that the identified error has been resolved. In this regard, a dose order with an error is not introduced in to the dose order queue.

Managing an error of a dose order record includes the pharmacy workflow application 114 accessing 1004 a dose order record corresponding to a dose order stored in, for example, local database 112. The dose order record may correspond to a dose order received by the pharmacy workflow application 114 that has not yet been prepared. Furthermore, the dose order may include a request for medication that is awaiting preparation in the pharmacy workflow application 114. In some instances, the corresponding dose order record of the dose order may be stored in a queue comprised of a plurality of dose order records with corresponding dose orders also awaiting preparation. In either event, in this embodiment, the pharmacy workflow application accesses a single dose record.

The pharmacy workflow application 114 may execute a first processing 1008 sequence in order to generate an alert corresponding to an error state related to the dose order. The error state, as will be discussed in more detail below, may correspond to a plurality of potential errors of the dose order records. The potential errors may include, for example, parsing errors, unknown unit errors, unknown product errors, unknown route errors, and the like. The first processing 1008 may include attempting to initiate a dose preparation protocol such upon initial of the dose preparation protocol the pharmacy workflow application identifies the error state.

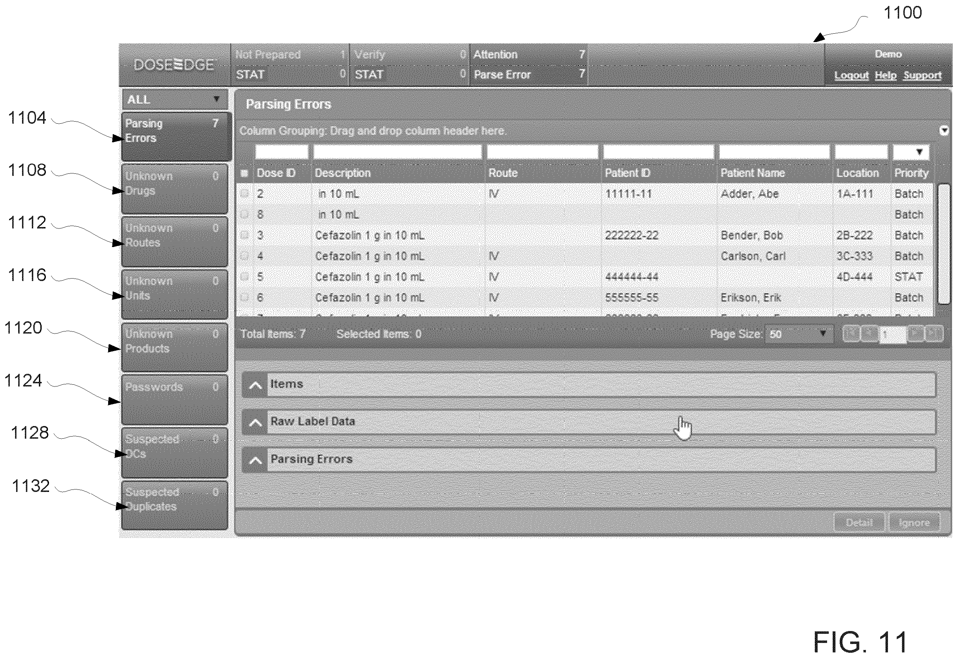

The previously noted interface 108 of local system 110 may also be operable to display 1012 a dose order in corresponding relation to the alert. That is, for example, the dose order may be displayed to the user in a manner that communicates to the user that the dose order is associated with an error state. The displayed dose order associated with the error state may, in some instances, be displayed with a plurality of other dose orders also associated an error state. The plurality of dose order associated with an error state may be grouped according to error type via one or more tabs, and highlighted with a color indicative of the error state. In this regard, the user may view a queue of dose orders such that each dose order of the queue is associated with an error state. The ability to view a plurality of dose orders in a queue in this manner may facilitate resolution of errors across the plurality of dose orders associated with errors. In some instances this queue may be dispose in an "Attention Tab," as depicted in Fig. 11, of the pharmacy workflow application 114, as discussed in more detail below.

The Attention Tab may facilitate the resolution of the errors associated with the dose order via Attention Tab user interface 1100. In particular, the pharmacy workflow application 114 may be operable to receive 1016 an input from a user indicative of a resolution of an error. For example, the input may correspond to a user-entered route to resolve an error of a dose order associated with an unknown route. In this regard, the Attention Tab 1100 may provide the user with the ability to remedy the error of the dose order record.

Furthermore, the pharmacy workflow application 114, via the attention tab 1100, may facilitate a second processing 1020 of the dose order based on the received input. In this regard, the pharmacy workflow application 114 may verify that the error of the dose order record has been appropriately resolved. For example, a user-entered route may be verified to ensure that that dose order record is no longer associated with an unknown route error. The unknown route error may still exist, for example, where the user entered the wrong information for the route. As such, the second processing 1020 may help ensure that a dose transferred from the Attention Tab 1100 is not subsequently sent back to the attention tab 1100 with additional errors - *i.e.,* only error-free dose order records may leave the attention tab. In this regard, the pharmacy workflow application 114 may requeue 1024 the dose order record upon confirmation of the successful resolution of the error of the dose order record.

As noted, the pharmacy workflow application 114 may be operable to generate an alert corresponding to a plurality of error states related to the dose order. For the sake of illustration, several error states associated with the foregoing attention tab 1100 are presented below. It will be appreciated that the several error states of the attention tab 1100 are not limiting, and that the pharmacy workflow application 114 may be operable to generate an alert corresponding to a plurality of other error states of the dose order record.

In this regard, the pharmacy workflow application 114 may be operable to generate an alert corresponding to an error state related to a parsing error. A dose order record associated with a parsing error may be disposed on a parsing errors tab 1104 of the attention tab 1100. A parsing error may include an error where, as a result of extracting the dose order from an independent data stream (*e.g*., an HL7 data stream), various aspects of the alpha numeric characters became unintelligible. In other instances, a parsing error may correspond to a dose order with incorrectly inputted alpha numeric characters at the time of creating the dose order - *i.e.,* misspelling the name of a formulary drug product. In any event, a parsing error results at least when the pharmacy workflow application 114 cannot recognize one or data fields or categories of the dose order record (*e*.*g*., the pharmacy workflow application 114 cannot determine which portion of a string of alpha numeric characters corresponds to a drug field - the drug field cannot be identified by reference to the string). This contrasts with other error types, discussed in greater below, such an unknown drug error, where the pharmacy workflow application 114 may recognize the drug field, but not the contents of the field. Upon generating an alert corresponding to the parsing error, the user may view the parsing error.

In another aspect, the pharmacy workflow application 114 may be operable to generate an alert corresponding to an error state related to an unknown drug error. A dose order record associated with an unknown drug error may be disposed on an unknown drug error tab 1108 of the attention tab 1100. In this regard, the pharmacy workflow application 114 may identify a drug field for the dose order with an unrecognized value of data. As such, an unknown drug error may include an error where the drug type associated with the dose order record is unrecognizable by the pharmacy workflow application 114. The determination of the unknown drug error may be accomplished with reference to a formulary database comprised of a plurality of formulary data records corresponding to various products. If the drug type associated with the dose order record is not related to a similar formulary data record, the pharmacy workflow application 114 may generate an alert corresponding to the error state of the unknown drug error. Upon generating an alert corresponding to the unknown drug error, the user may view the unknown drug error or take steps resolute the error. For example, the user may update the formulary record database by creating a new product definition consistent with the product information of the dose order record. In this regard, upon second processing, the pharmacy workflow application 114 may identify the newly created formulary record such that the dose order record is no longer associated with an unknown drug *- i.e.,* the error was resolved.

In this regard, the pharmacy workflow application 114 may be operable to generate an alert corresponding to an error state related to an unknown route error. A dose order record associated with an unknown route may be disposed on an unknown route tab 1112 of the attention tab 1100. In this regard, the pharmacy workflow application 114 may identify a route field for the dose order with an unrecognized value of data. As such, an unknown route error may include an error where the route type (*i.e.,* how the drug is administered to a patient) associated with the dose order record is unrecognizable by the pharmacy workflow application 114. The determination of the unknown route error may be accomplished with reference to the formulary database. If the route type associated with the dose order record is not related to a similar formulary data record, the pharmacy workflow application 114 may generate an alert corresponding to the error state of the unknown route error. Upon generating an alert corresponding to the unknown route error, the user may view the unknown route error or take steps resolute the error. For example, the user may update the formulary record database by creating a new route definition in an existing formulary record consistent with the route information of the dose order record. In this regard, upon second processing, the pharmacy workflow application 114 may identify the newly updated formulary record that contains the new route such that the dose order record is no longer associated with an unknown drug *- i.e.,* the error was resolved.

In this regard, the pharmacy workflow application 114 may be operable to generate an alert corresponding to an error state related to an unknown unit error. A dose order record associated with an unknown unit error may be disposed on an unknown unit errors tab 1116 of the attention tab 1100. In this regard, the pharmacy workflow application 114 may identify a unit field for the dose order with an unrecognized value of data. As such, an unknown unit error may include an error where the unit type (*i.e.,* the unit of measurement of the various products of the dose order) associated with the dose order record is unrecognizable by the pharmacy workflow application 114. Upon generating an alert corresponding to the parsing error, the user may view the unknown unit error.

In this regard, the pharmacy workflow application 114 may be operable to generate an alert corresponding to an error state related to an unknown products error. A dose order record associated with an unknown products error may be disposed on an unknown products errors tab 1120 of the attention tab 1100. In this regard, the pharmacy workflow application 114 may identify a products field for the dose order with an unrecognized value of data. As such, an unknown products error may include an error where the product type (*e*.*g*., a diluent) associated with the dose order record is unrecognizable by the pharmacy workflow application 114. The determination of the unknown product error may be accomplished with reference to a formulary database comprise of a plurality of formulary data records corresponding to various products. If the product type associated with the dose order record is not related to a similar formulary data record, the pharmacy workflow application 114 may generate an alert corresponding to the error state of the unknown product error. Upon generating an alert corresponding to the unknown drug error, the user may view the unknown drug error or take steps resolute the error. For example, the user may update the formulary record database by creating a new product definition consistent with the product information of the dose order record. In this regard, upon second processing, the pharmacy workflow application 114 may identify the newly created formulary record such that the dose order record is no longer associated with an unknown product - *i.e.,* the error was resolved.

Other critical issues may be addressed by the pharmacy workflow management application 114 via the attention tab 110. For example, the pharmacy workflow application 114 may be operable to generate an alert corresponding to an error state related to a passwords error. A dose order record associated with a passwords error may be disposed on a passwords errors tab 1124 of the attention tab 1100. A passwords error may include an error where a user account password is expired or where a user account is disable. An administrator-level user may view alerts associated with used accounts with expired passwords or that are otherwise disabled. In some embodiments, the pharmacy workflow application 114 may accept an input from an administrator-level user indicative of a resolution of the expired password alert or the account disablement alert.

The pharmacy workflow application 114 may also be operable to generate an alert corresponding to an error state related to a suspected discontinued dose error. A dose order record associated with a suspected discontinued dose error may be disposed on a suspected discontinued dose errors tab 1128 of the attention tab 1100. A suspected discontinued dose error may include an error where the dose order record is suspected of being associated with a discontinued dose order. For example, the previously inputted dose order may no longer be required in the hospital setting in which it was originally order. As such, the dose order may be cancelled or discontinued. According, the pharmacy workflow application may 114 may associate an alert with the identified suspected discontinued dose order. Upon generating an alert corresponding to the suspected discontinued dose error, the user may view the suspected discontinued dose error. In some embodiments, the pharmacy workflow application 114 may be operable to delete the suspected discontinued dose order in response to a user input indicative of a resolution of the suspected discontinued dose error.

In this regard, the pharmacy workflow application 114 may be operable to generate an alert corresponding to an error state related to a suspected duplicated error. A dose order record associated with a suspected duplicated error may be disposed on a suspected duplicated errors tab 1132 of the attention tab 1100. A suspected duplicated error may include an error where the where the dose order record is suspected of being associated with a duplicate dose order. For example, a dose order may have inadvertently been inputted into the system twice. In such an instance, the duplicate dose order is not required and need not be prepared. According, the pharmacy workflow application may 114 may associate an alert with the identified suspected duplicate dose order. Upon generating an alert corresponding to the suspected duplicate dose error, the user may view the suspected duplicate dose error. In some embodiments, the pharmacy workflow application 114 may be operable to delete the suspected duplicate dose order in response to a user input indicative of a resolution of the suspected duplicate dose error.

In this regard, the pharmacy workspace application 114 may provide functionality associated with identifying suspect duplicate dose order and suspect discontinued dose orders as described in U.S. Provisional Patent Application No. 61/975,519 entitled "MANAGEMENT OF MEDICATION DOSE ORDERS" filed on April 4, 2014, which is co-owned with the present application. In this regard, the pharmacy workflow application 114 may execute a duplicate order detection rule, such that the pharmacy workflow application 114 may be operative to review a first dose order record in view of other dose order records within the dose order queue to determine if the first dose order record corresponds to a potential duplicate dose order. In this regard, the pharmacy workflow application 114 may evaluate the metadata regarding the first dose order record to determine if other ones of the dose order records within the dose order queue match the identified metadata regarding the dose order to a predetermined correspondence. In some instances, the predetermined correspondence may be selectable by a user. It may be appreciated that the matching of the metadata between the dose order record being reviewed in the other dose order records in the queue may not necessarily need to be identical. For example, the rule may be written such that if selected ones of the metadata fields are identical between the first dose order record being reviewed and the other dose order records in the dose order queue, the dose order record being reviewed may be flagged is a potential duplicate order. Such a rule may be applied to the first dose order record when the record is initially populated the dose order queue.

In other embodiments, the pharmacy workflow application 114 may process incoming dose orders to determine if the order corresponds to a discontinuation of an order. That is, a common practice when processing dose orders is to indicate a discontinuation of a first order by subsequently sending a corresponding order to the first order at a later time with a discontinue status for the subsequently provided order. In prior approaches, the receipt of such a discontinued order may simply result in printing a label with the dose order details and a discontinuation status. This would in turn require a user to go through the printed dose order labels to locate the original dose order that was referenced in the discontinued dose order in order to replace the original dose order on a discontinued status. The pharmacy workflow application 114 may be utilized to automate this process pharmacy workflow application 114 by identifying receipt of a dose order in the dose order queue that contains a discontinued status and automatically matching the discontinued dose to one or more existing dose order records. Thus, receipt of a dose order with a discontinued status may trigger the pharmacy workflow application 114 to perform a query of the dose order queue to identify corresponding ones of the dose orders in the dose order queue that exceed a predetermined correspondence to the discontinued dose order. For example, one or more overlapping or identical pieces of metadata between the discontinue dose order and the identified discontinued order in the dose order queue may be determined. The predetermined correspondence may be customizable by a user. In any regard, upon identification of a dose order record from the dose order queue that corresponds to a received discontinued dose order, the original dose order may automatically change the status of the one or more identified dose order records to a discontinued status.

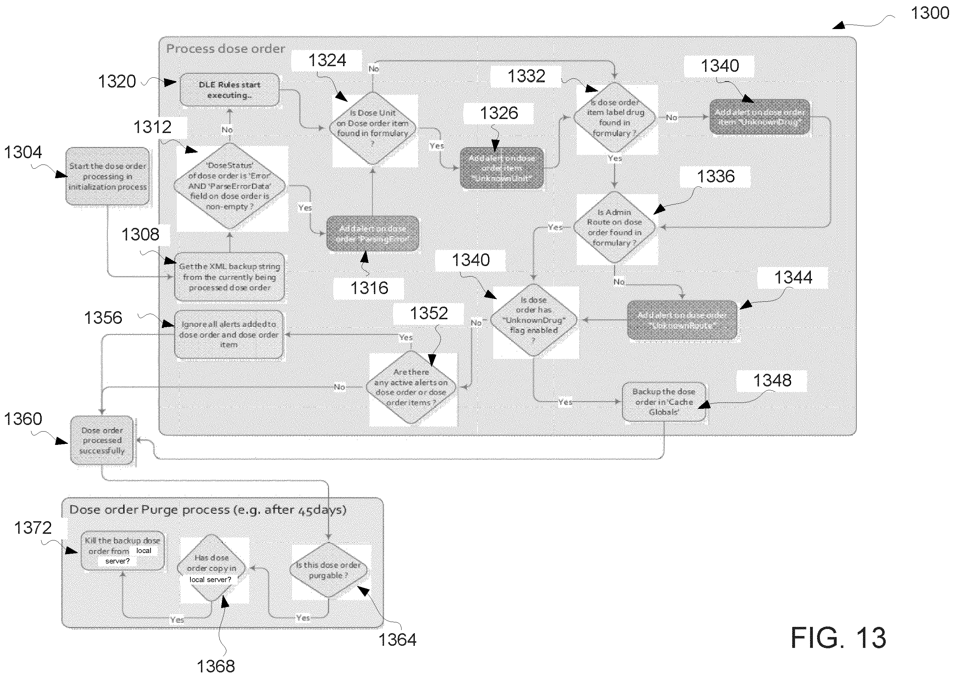

Turning next to Fig. 13, the pharmacy workflow application 114 may facilitate the identification of alerts on a dose order of the dose record according generally to method 1300. Method 1300 may proceed by initiating the dose order initialization process 1304. This may correspond to the pharmacy workflow application 114 initiating the dose preparation protocol corresponding to the dose order. Next, the pharmacy workflow application 114 may obtain the XML backup string from the currently processed dose order 1308. The method 1500 may continue at step 1312 such that the dose status of the dose order is error and parse error data field on the dose order is non-empty. To the extent that the dose status of the dose order comprises an error value and the parse error data field of the dose order is nonempty, the method 1500 may continue at step 1316 such that pharmacy workflow application 114 adds an alert to the dose order indicative of a parsing error. However, to the extent that either the dose status of the dose order dose not comprise an error, or the parse error data field of the dose order is empty, the method 1300 may continue at step 1320 such that the dose logic engine ("DLE") initiates the dose logic engine execution sequence.

In this regard, regardless of whether the dose status of the dose order comprises an error, or the parse error data field of the dose order is nonempty, the method 1300 may proceed to step 1324 such that dose unit of the dose order is identified in the formulary. To the extent that the dose unit of the dose order is not identifiable in the formulary, the method 1300 may proceed to step 1328 such that pharmacy workspace application 114 adds an alert to the dose order record indicative of an unknown unit error. To the extent that a dose unit of the dose order is identifiable in the formulary, or the pharmacy workflow application 114 added an alert to a dose order item indicative of an unknown unit error, the method of 1300 may proceed to step 1332, such that pharmacy workflow application 114 may determine whether the dose drug of the dose order is identified in the formulary. To the extent that the dose drug of the dose order is not identified in the formulary, the method 1500 may continue to step 1340 such that pharmacy workflow application 114 adds an alert to the dose order associated with an unknown drug error.

To the extent that the dose drug of the dose order is identified in the formulary, or the pharmacy workflow application 114 added an alert to the dose order indicative of an unknown drug, the method 1300 may continue at step 1336 such that the pharmacy workflow application 114 may determine if the administration route of the dose order is found in the formulary. To the extent that the administration route of the dose order is not found in the formulary, the method 1300 may continue at step 1344 such that pharmacy workflow application 114 adds an alert to the dose order indicative of an unknown route. To the extent that an administration route of the dose order is found in the formulary, or the pharmacy workflow application 114 added an alert to the dose order indicative of an unknown route, the method 1300 may proceed at step 1340 such that the pharmacy workflow application 114 determines if a dose order includes an unknown drug flag. If the dose order is includes an unknown drug flag, the method 1300 may proceed to step 1348 such that the pharmacy workflow application 114 may backup the dose order in a local server.

In this regard, method 1300 may proceed to step 1360 such that the dose order is successfully processed. If, however, the dose order is not enabled with the unknown drug flag, the method 1300 may continue at step 1352 such that pharmacy workflow application 114 determines whether there are any active alerts on the dose order or associated aspects of the dose order record. To the extent that there are active alerts on the dose order or dose order record, the method 1300 may continue at step 1356 such that all alerts added to the dose order or dose order record may be ignored. Regardless of whether there are any active alert on the dose order or dose order record, the method 1300 may continue at step 1360 such that the pharmacy workflow application 114 successfully processes the dose order.

In this regard, method 1300 may facilitate executing a dose order purge process *(e.g.,* after 45 days). In this regard, the method 1300 may continue at step 1364 such that the pharmacy workflow application 114 determines whether the dose order is purgeable. If the dose order is purgeable, the method of 1300 may continue at step 1368 such that the pharmacy workflow application 114 determines whether a dose order copy is stored in the local server. If the dose order copy is stored in the local server, the pharmacy workflow application 114 may remove the backup dose order from the local server.

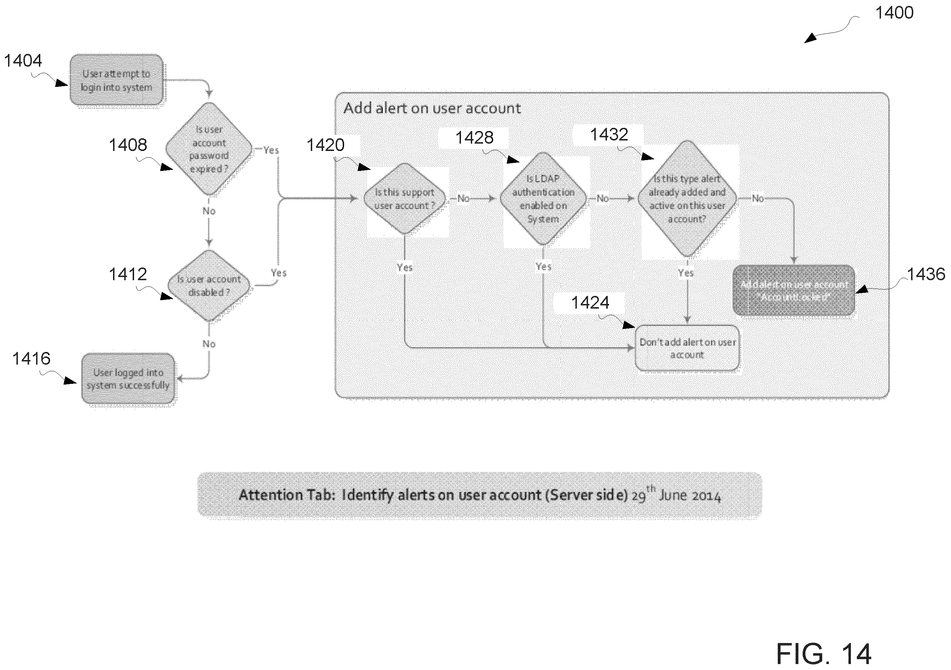

Turning next to Fig. 14, the pharmacy workflow application 114 may facilitate the identification of alerts on a user account according generally to method 1400. Method 1400 may proceed upon the initiation of step 1404 such that the pharmacy workflow application 114 identifies an attempt by a user to log in to the pharmacy workflow application 114 via user interface 108, for example. The method 1400 may continue at step 1408 such that the pharmacy workflow application determines whether the password associated with the user account is expired. To the extent that the password associated with the user account is not expired, the method 1400 may continue at step 1412 such that the pharmacy workflow application 114 determines if the user account has been disabled in any regard. To the extent, that the user account has not been disabled, the method 1400 may continue at step 1416 such that the pharmacy workflow application 114 determines that the user successfully logged in to the pharmacy workflow application 114.

If, however, the password associated with the user account has been disabled, or the user account has otherwise been disabled, the method 1400 may continue at step 1420 such that the pharmacy workflow application 114 determines if the user account is a support user account. To the extent that the user account is a support user account, the method 1400 may proceed to step 1424 such that pharmacy workflow application 114 does not add any alert on the user account. To the extent that the user account is not a support user account, the method 1400 may continue at step 1428 such that the pharmacy workflow application may determine if LDAP authentication is activated within the pharmacy workflow application 114. To the extent that LDAP authentication is activated within the pharmacy workflow application 114, the method 1400 may continue at step 1424 such that the pharmacy workflow application 114 does not add an alert to the user account. However to the extent that LDAP authentication is not activated within the pharmacy workflow application 114, the method 1400 may continue at step 1432 such that the pharmacy workflow application 114 determines if an alert of the same type has previously been added and active on this user account. To the extent that in alert of the same type has been previously added an active on this user account, the method 1400 may continue at step 1424 such that the pharmacy workflow application does not add an alert to the user account. However, to the extent that new alert of the same type has not been previously added and active on this user account, the method 1400 may continue at step 1436 such that the pharmacy workflow application 114 may at any alert on the user account indicative of in account locked status.

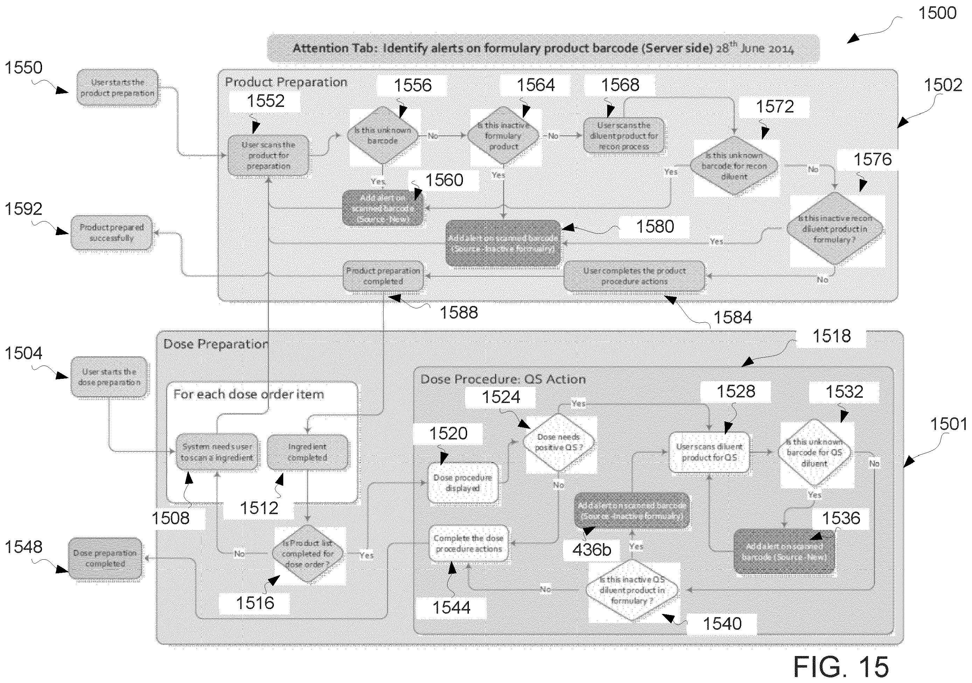

Turning next to Fig. 15, the pharmacy workflow application 114 may facilitate identification of alerts on scanned product barcodes according generally to a method 1500. Method 1500 may be comprised generally of steps associated with dose preparation 1501 and steps associated with product preparation 1502. In this regard method 1500 may begin at step 1504 such that the dose preparation is initiated. The method may continue according generally to dose preparation 1501 such that the method 1500 continues at step 1508 such that the pharmacy workflow application 114 prompts a user to scan a product. Notably, step 1508 may initiate the steps associated with product preparation steps 1502, discussed in greater detail below. The completion of steps generally associated with product preparation 1502 may facilitate the method 1500 to continue at step 1512 such that the product ingredient preparation is complete. The method 1500 may continue at step 1516 such that the pharmacy workflow application 114 determines whether the product list is completed for the dose order. To the extent that the product list is not completed for the dose order, the pharmacy workflow application 114 may return to step 1508 such that the pharmacy workflow application 114 prompts a user to scan a product. If, however, the product list is complete for the dose order, the method 1500 may continue by executing the steps generally associated with a dose procedure QS action 1518.

In this regard, the method 1500 may continue at step 1520 such that the dose procedure is displayed. The method 1500 may continue at step 1524 such that the pharmacy workflow application 114 determines whether the dose order requires a positive QS *(e.g.,* diluent product). To the extent that the dose order requires a positive QS, the method 1500 may continue at step 1528 such that the user scans a diluent product for subsequent QS. The method 1500 may continue at step 1532 such that the pharmacy workflow application 114 determines whether the scanned diluent product is associated with an unknown barcode. To the extent that the scanned diluent product is associated with an unknown barcode, the method 1500 may proceed at step 1536 such that the pharmacy workflow application 114 may add an alert associated with the scanned diluent product indicative of a new source product.

In this regard, the method 1500 may proceed with returned reference to step 1528 such that a user scans a diluent product for a subsequent QS determination. With returned reference to step 1532, to the extent that the scanned diluent product is not associated with an unknown barcode, the method 1500 may proceed to step 1540 such that the pharmacy workflow application 114 determines whether the scanned diluent product is associated with an inactive QS diluent product in the formulary. To the extent that the scanned diluent product is associated with an inactive QS diluent product in the formulary, the method 1500 may proceed to step 1544 such that the pharmacy workflow application 114 may add an alert associated with the scanned diluent product indicative of an inactive formulary product.

In this regard, the method 1500 may proceed with returned reference to step 1528 such that a user scans a diluent product for a subsequent QS determination. To the extent that the scanned diluent product is not associated with inactive QS diluent product in the formulary, the method 1500 may continue at step 1544 such that the dose procedure actions are completed. Notably, and with returned reference to step 1524 of method 1500, if the dose does not require a positive QS, the method 1500 may proceed to step 1544 too. In this regard, the method 1500 may proceed to step 1548 such that the dose preparation is complete.

With returned reference to step 1508, the method 1500 may generally execute the steps associated with the product preparation 1502 such that a product may be scanned for product preparation at step 1552 after pharmacy workflow application 114 prompts a user to scan a product at step 1508. In some embodiments, step 1552 may initiate after a user starts the product preparation at step 1550. The method 1500 may continue at step 1556 such that the pharmacy workflow application 114 determines whether the barcode associated with the scan product is unknown. If the barcode associated with the scan product is unknown, the method 1500 may continue at step 1560 such that pharmacy workflow application 114 adds an alert associated with the barcode of the scanned product indicative of a new product.

In this regard, the method 1500 may continue with returned reference to step 1552 such that a product may be scanned for product preparation. To the extent that the barcode associated with the scanned product is not unknown, the method 1500 may continue at step 1564 such that pharmacy workflow application 114 may determine whether the scanned product is in an active formulary product. To the extent of the scan product is an inactive formulary product, the method 1500 may proceed to step 1580 such that pharmacy workflow application 114 may add an alert associated with the barcode of the scanned product indicative of an inactive formulary product.

In this regard, the method 1500 may continue with returned reference to step 1552 such that a product may be scanned for product preparation. If, however, the scanned product is not associated with an inactive formulary product, the method 1500 may continue at step 1568 such that the user scans a diluent product for a reconstitution process. The method 1500 may continue at step 1572 such that pharmacy workflow application 114 determines if the barcode of the scanned reconstitution diluent product is unknown. To the extent that the barcode of the scanned reconstitution diluent product is unknown, the method 1500 may continue with returned reference to step 1560 such that pharmacy workflow application 114 may add an alert associated with the barcode of the scanned reconstitution diluent product indicative of a new product. If, however, the barcode of the scanned reconstitution diluent product is not unknown, the method 1500 may continue at step 1576 such that pharmacy workflow application 114 may determine if the barcode of the scanned reconstitution diluent product is inactive. To the extent that the barcode of the scanned reconstitution diluent product is inactive, the method 1500 may continue with returned reference to step 1580 such that the pharmacy workflow application 114 may add an alert associated with the barcode of the scanned reconstitution diluent product indicative of an inactive formulary product. If, however, the barcode of the scanned reconstitution diluent product is not inactive, the method 1500 may continue at step 1584 such that the user may complete the product procedure actions. The method 1500 may continue at step 1588 such that the product preparation is complete. In this regard, the method 1500 may continue at step 1592 such that the pharmacy workflow application 114 indicates successful product preparation. Notably, the completion of step 1588 may facilitate the continuation of method 1500 at step 1512 such that the product ingredient preparation is complete.

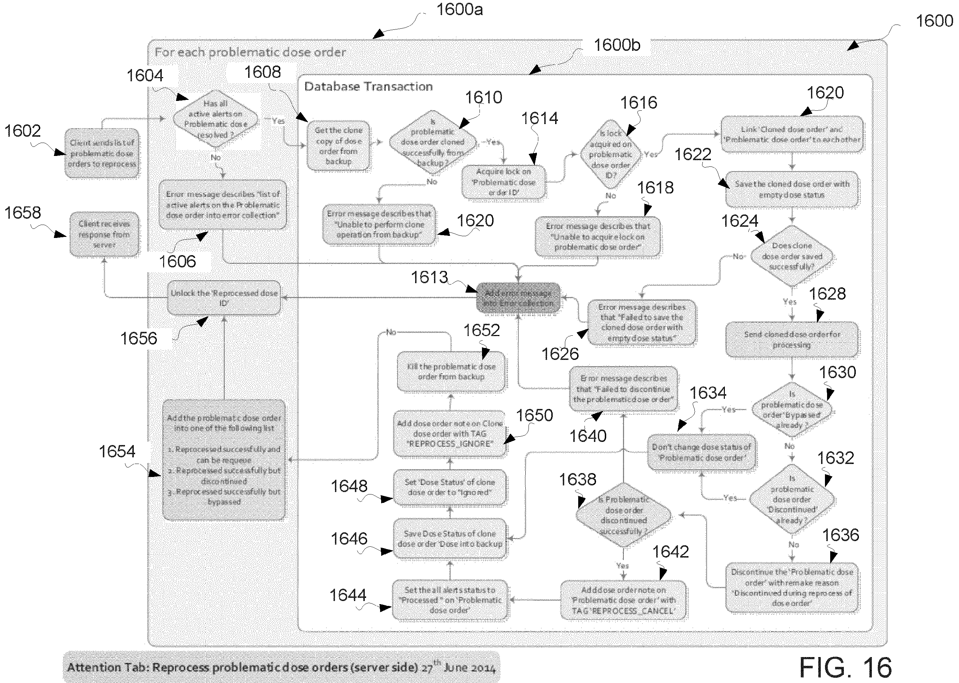

Turning next to Fig. 16, the pharmacy workflow application 114 may facilitate the reprocessing of dose orders according generally to method 1600. Method 1600 may generally comprise steps associated with actions taken for each problematic dose order 1600a and steps associated with database transactions 1600b. In this regard, method 1600 may begin at step 1602 such pharmacy workflow application 114 may receive a list of problematic dose orders for reprocessing according to method 1600. The method 1600 may continue at step 1604 such that pharmacy workflow application 114 may determine if all active alerts of the problematic dose order have been resolved. To the extent that all active alerts of the problematic dose order have not been resolved, method 1600 may continue at step 1606 such that the pharmacy workflow application 114 may generate an error message, which notes: "list of active alerts of the problematic dose order into error collection."

In this regard, the method 1600 may continue at step 1613 such that pharmacy flow application 114 may add the error message into in error collection associated with the dose order. If, however, the pharmacy workspace application 114 determines, with returned reference to step 1604, that all active alerts of the problematic dose order have been resolved, the method 1600 may continue by executing the steps generally associated with database transaction 1600b. In this regard, the method 1600 may continue at step 1608 such that the pharmacy workflow application 114 obtains a cloned copy of the dose order from a backup. The method 1600 may continue at step 1610 such that the pharmacy workflow application 114 determines if the problematic dose order has been successfully cloned from the backup. To the extent that the pharmacy workflow application 114 does not successfully obtain a clone from the backup, the method 1600 may continue at step 1612 such that the pharmacy workflow application 114 may generate an error message, which notes: "unable to perform clone operation from backup." In this regard, method 1600 may continue at step 1613 according to the discussion above.

If, however, the pharmacy workflow application 114 does successfully obtain a clone from the backup, the method 1600 may continue at step 1614 such that the pharmacy workflow application 114 acquires a lock on the problematic dose order ID. The method 1600 may continue at step 1616 such that the pharmacy workflow application 114 determines whether a lock has been acquired on the problematic dose order ID. To the extent that a lock has not been acquired on the problematic dose order ID, the method the 1600 may continue at step 1618 such that the pharmacy workflow application 114 may generate an error message, which notes: "unable to acquire lock on problematic dose order." In this regard, method 1600 may continue at step 1613 according to the discussion above.

If, however, a lock is acquired on the problematic dose order ID, the method 1600 may continue at step 1620 such that the cloned dose order and problematic dose order may be linked to each other. The method 1600 may continue at step 1622 such that the cloned dose order is saved with an empty dose order status. Method 1600 may continue at step 1624 such that the pharmacy workflow application 114 may determine whether the cloned dose order has been saved successfully. To the extent that the cloned dose order has not been saved successfully, the pharmacy workflow application 114 may generate an error message, which notes: "failed to save the cloned dose order with empty dose status." In this regard, method 1600 may continue at step 1613 according to the discussion above.

If, however, the cloned dose order has been saved successfully, the method 1600 may continue at step 1628 such that he cloned dose order is sent for processing. The method 1600 may continue at step 1630 such that the pharmacy workflow application 114 may determine whether the problematic dose order has already been bypassed. To the extent that the problematic dose order has not already been bypassed, the method 1600 may proceed to step 1632 such that the pharmacy workflow application 114 may determine whether the problematic dose order is already discontinued. If the problematic dose order has already been bypassed or the problematic dose order is already discontinued, the method 1600 may continue at step 1634 such that the status of the problematic dose order is unchanged. The pharmacy work flow application 114 may save the dose status of the cloned dose order to a backup file.

With returned reference to step 1634, if the problematic dose order is not already discontinued, the method 1600 may continue at step 1636 such that the pharmacy workflow application 114 may discontinue the problematic dose order with a remake reason, which notes: "discontinue during reprocess of dose order." The method 1600 may continue at step 1638 such that the pharmacy workflow application 114 may determine whether the problematic dose order has been successfully discontinued. To the extent that the problematic dose order has not been successfully discontinued, the method 1600 may continue at step 1640 such that the pharmacy workflow application 114 may generate an error message, which notes: "failed to discontinue the problematic dose order." If, however, the problematic dose order has been successfully discontinued the method 1600 may proceed to step 1642 such that the pharmacy workflow application 114 may add a dose order note associated with the problematic dose order with the tag: "reprocess_cancel."

In another aspect, the method 1600 may continue at step 1644 such that the pharmacy workflow application 114 makes the all alert status of the problematic dose order set to: "processed." The method 1600 may continue at step 1646 such that the pharmacy workflow application 114 may save the dose order status of the cloned dose order to a backup file. The method 1600 may continue at step 1648 such that the pharmacy workflow application 114 may set the dose status of the cloned dose order to "ignore." The method 1600 may continue at step 1650 such that pharmacy workflow application 114 may add a dose order note associated with the cloned dose order with the tag: "reprocess_ignore." The method 1600 may continue at step 1652 such that the pharmacy workflow application 114 may remove the problematic dose order from the backup.

In this regard, the steps associated with database transaction 1600b may be substantially performed. The method 1600 may continue at step 1654 such that the pharmacy workflow application 114 may add the problematic dose order into one or more of the following lists: reprocessed successfully and can be requeued, reprocessed successfully but discontinued, and reprocessed successfully but bypassed. The method 1600 may continue at step 1656 such that pharmacy workflow application 114 may unlock the reprocessed dose ID. Notably, step 1613 of method 1600, discussed in greater detail above, may proceed to step 1656. The method may continue at step 1658 such that the client device may receive a response from the pharmacy workflow application 114 associated with the resolution of the problematic dose order.

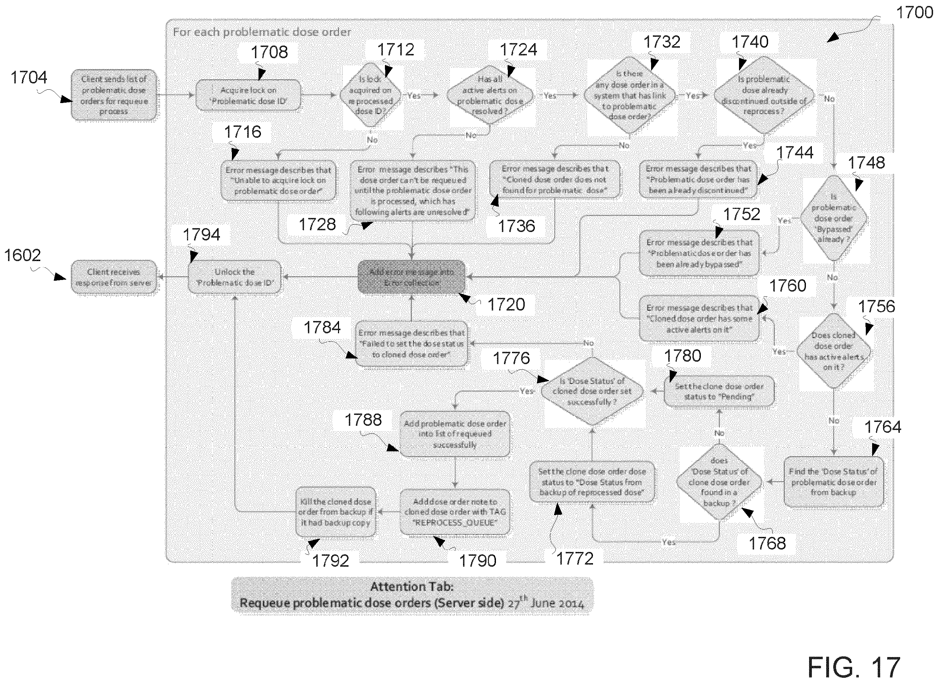

Turning next to Fig. 17, the pharmacy work flow application 114 may facilitate the requeue of dose orders. In this regard, the method 1700 may begin at step 1704 such that the pharmacy workflow application 114 receives a list of problematic dose orders for the requeue process. The method 1700 may continue at step 1708 such that the pharmacy workflow application 114 acquires a lock on the problematic dose ID. The method 1700 may continue at step 1712 such that the pharmacy workflow application determines if a lock has been acquired on the reprocessed dose IDs. To the extent that a lock has not been acquired on the reprocessed dose IDs, the method 1700 may continue and step 1716 such that the pharmacy workflow application 114 may generate an error message, which notes: "unable to require lock on problematic dose order." In this regard, the method 1700 may continue at step 1720 such that the pharmacy workflow application 114 adds an error message to an error collection of the problematic dose order. To the extent that the lock is acquired on the reprocessed dose ID, the method 1700 may continue at step 1724 such that the pharmacy workflow application 114 may determine if all active alerts of the problematic dose order have been resolved. If all active alert on the problematic dose order have not been resolved, the method 1700 may continue at step 1728 such that the pharmacy workflow application 114 may generate an error message, which notes: "this does order can't be requeued until the problematic dose order is processed, which has following alerts that are unresolved." In this regard, the method 1700 may continue at step 1720 as described above.

If, however, all active alerts on, or otherwise associated with, the problematic dose order have been resolved, the method 1700 may continue at step 1732 such that the pharmacy workflow application 114 may determine whether any other dose orders are linked to the problematic dose order. To the extent that there are not any dose orders in the system that are linked to the problematic dose order, the method 1700 may continue at step 1736 such that the pharmacy workflow application 114 may generate an error message, which notes: "cloned dose order is not found for the problematic dose." In this regard, the method 1700 may continue at step 1720 as described above.

To the extent that there are dose orders in the system that are linked to the problematic dose order, the method 1700 may continue at step 1740 such that the pharmacy workflow application 114 determines whether the problematic dose order has already been discontinued outside of the reprocess functionality. If the problematic dose order has already been discontinued outside of the reprocess functionality, the method 1700 may continue at step 1744 such that the pharmacy workflow application 114 may generate an error message, which notes: "problematic dose order has already been discontinued." In this regard, the method 1700 may continue at step 1720 as described above.

If, however, the problematic dose order has not already been discontinued outside of the reprocess functionality, the method 1700 may continue at step 1748 such that the pharmacy workflow application 114 may determine whether the problematic dose order has already been bypassed. If the problematic dose order has not already been bypassed, the method 1700 may continue at step 1756 such that the pharmacy workflow application 114 may determine whether the cloned dose order has any active alerts associated with it. In this regard, if the problematic dose order has already been bypassed, or the cloned dose order has active alerts associated with it, the method 1700 may proceed to step 1752 and step 1760, respectively. That is, if the problematic dose order has already been bypassed, the method 1700 may proceed to step 1752 such that the pharmacy workflow application 114 may generate an error message that notes: "problematic dose order has already been bypassed." And, if the cloned dose order has active alerts associated with it, the method 1700 may continue at step 1760 such that the pharmacy work flow application 114 may generate an error message, which notes: "cloned dose order has some active alerts associated with it." In response to either step 1752 or step 1760, the method 1700 may continue at step 1720 as discussed above.

With returned reference to step 1756, to the extent that the cloned dose order does not have any active alerts associated with it, the method 1700 may continue at step 1764 such that the pharmacy workflow application 114 may find the dose status of the problematic dose order from the backup. The method 1700 may continue at step 1768 such that the pharmacy workflow application 114 may determine whether the dose status of the cloned dose order is found in the backup. To the extent that the dose status of the cloned dose order is not found in the backup, the method 1700 may continue at step 1780 such that the pharmacy workflow application 114 may set the cloned dose order status to pending. If, however, the dose status of the cloned dose order is found in the backup, the method 1700 may continue at step 1772 such that the pharmacy workflow application 114 may set the cloned dose order dose status to: "dose status from backup of reprocess dose." In response to the completion of either step 1772 or 1780, the method 1700 may continue at step 1776 such that the pharmacy workflow application 114 may determine whether the dose status of the cloned dose order has been successfully set. To the extent that the dose status of the cloned dose order has not been successfully set, the method 1700 may continue at step 1784 such that the pharmacy workflow application 114 may generate an error message, which notes: "failed to set the dose status as to cloned dose order." In this regard, the method 1700 may continue at step 1720 as described above.

If, however, the dose status of the cloned dose order has been successfully set, the method 1700 may continue at step 1788 such that the pharmacy workflow application 114 may add the problematic dose order into a list of successfully requeued doses. The method 1700 may continue at step 1790 such that the pharmacy workflow application 114 may add a dose order note to the cloned dose order with the tag: "reprocess_queue." The method 1700 may continue at step 1792 such that the pharmacy workflow application 114 may remove the cloned dose order from the backup in the case that the cloned dose order was associated with a back. The method 1700 may continue at step 1794 such that the pharmacy workflow application 114 may unlock the problematic dose ID. Notably, the completion of previously described steps 1720, may also facilitate the method 1700 continuing at step 1794. The method 1700 may continue at step 1796 such that the client receives a response from the pharmacy workflow application 114.

## Claims

1. A system (100) for batch processing of a plurality of dose orders for preparing corresponding compounded medication doses, the system comprising:
a first database (112) storing a plurality of different dose preparation protocols for associated compounded medication doses, each of the dose preparation protocols specifying
a name of the associated compounded medication dose,
at least one drug product,
at least one diluent product, and
operations for verifying the at least one drug product, the at least one diluent product, and the associated compounded medication dose;
a second database (112) storing a plurality of dose order records, each dose order record specifying at least
the name of the compounded medication dose,
a volume of the at least one drug product,
a final volume of the compounded medication dose,
a local system (110) communicatively coupled to the first database and the second database, the local system (110) configured to
determine which of the plurality of dose order records include the same at least one drug product and the same at least one diluent product,
aggregate dose order records of the plurality of dose order records as a batch order for the dose order records that include the same at least one drug product and the same at least one diluent product,
select the dose preparation protocol from the first database that is associated with the aggregated dose order records,
modify the dose preparation protocol based on an amount of the aggregated dose order records by
determining a total volume of the at least one drug product by summing the volume of the at least one drug product that is specified by each of the aggregated dose order records,
determining a total volume of the compounded medication doses by summing the volume of the compounded medication dose that is specified by each of the aggregated dose order records,
determining a total volume of the at least one diluent product by subtracting the total volume of the at least one drug product from the total volume of the compounded medication doses, and
updating at least some of the operations based on the total volume of the at least one drug product, the total volume of the compounded medication doses, and the total volume of the at least one diluent product, and
create a batch workflow using the modified dose preparation protocol;
a pharmacy workflow management application (114) of the local system (110), the pharmacy workflow management application configured to
receive the batch workflow and the batch order from the local system (110),
cause at least one graphical user interface to display a first verification step for the total volume of the at least one drug product,
cause the at least one graphical user interface to display a second verification step for the total volume of the at least one diluent product,
cause the at least one graphical user interface to display a third verification step for the total volume of the compounded medication doses, and
cause the at least one graphical user interface to display a fourth verification step indicative that the compounded medication doses are provided in a separate container for each of the aggregated dose order records.

2. The system according to claim 1, wherein the pharmacy workflow management application is configured to cause labels to be printed for the separate containers.

3. The system according to anyone of claims 1 - 2, wherein the labels include a dose label or a work-in-progress label.

4. The system according to anyone of claims 1 - 3, wherein the at least one drug product includes a pharmaceutical drug or active medication ingredient for therapeutic effect.

5. The system according to anyone of claims 1 - 4, wherein each dose order record further includes a concentration of the at least one drug product, and wherein the local system (110) is configured to determine the total volume of the at least one diluent product using additionally the concentration of the at least one drug product.

## Patentansprüche

1. System (100) zur Batch-Verarbeitung einer Vielzahl von Dosisbestellungen zum Herstellen entsprechender zusammengesetzter Medikamentendosen, wobei das System Folgendes umfasst:
eine erste Datenbank (112), die eine Vielzahl von unterschiedlichen Dosisherstellungsprotokollen für zugehörige zusammengesetzte Medikamentendosen speichert, wobei jedes der Dosisherstellungsprotokolle Folgendes angibt
einen Namen der zugehörigen zusammengesetzten Medikamentendosis,
mindestens ein Arzneimittelprodukt,
mindestens ein Verdünnungsmittelprodukt, und
Betriebsvorgänge zum Verifizieren des mindestens einen Arzneimittelprodukts, des mindestens einen Verdünnungsmittelprodukts und der zugehörigen zusammengesetzten Medikamentendosis;
eine zweite Datenbank (112), die eine Vielzahl von Dosisbestellungsaufzeichnungen speichert, wobei jede Dosisbestellungsaufzeichnung mindestens Folgendes angibt
den Namen der zusammengesetzten Medikamentendosis,
ein Volumen des mindestens einen Arzneimittelprodukts,
ein Endvolumen der zusammengesetzten Medikamentendosis,
ein lokales System (110) in Kommunikationsverbindung mit der ersten Datenbank und der zweiten Datenbank, wobei das lokale System (110) so ausgestaltet ist, dass es
ermittelt, welche der Vielzahl von Dosisbestellungsaufzeichnungen das gleiche mindestens eine Arzneimittelprodukt und das gleiche mindestens eine Verdünnungsmittelprodukt umfassen,
Dosisbestellungsaufzeichnungen der Vielzahl von Dosisbestellungsaufzeichnungen als eine Batch-Bestellung für die Dosisbestellungsaufzeichnungen aggregiert, die das gleiche mindestens eine Arzneimittelprodukt und das gleiche mindestens eine Verdünnungsmittelprodukt umfasst,
das Dosisherstellungsprotokoll aus der ersten Datenbank auswählt, die mit den aggregierten Dosisbestellungsaufzeichnungen assoziiert ist,
das Dosisherstellungsprotokoll basierend auf einer Menge der aggregierten Dosisbestellungsaufzeichnungen modifiziert durch
Ermitteln eines Gesamtvolumens des mindestens einen Arzneimittelprodukts durch Summieren des Volumens des mindestens einen Arzneimittelprodukts, das durch jede der aggregierten Dosisbestellungsaufzeichnungen angegeben ist,
Ermitteln eines Gesamtvolumens der zusammengesetzten Medikamentendosen durch Summieren des Volumens der zusammengesetzten Medikamentendosis, das durch jede der aggregierten Dosisbestellungsaufzeichnungen angegeben ist,
Ermitteln eines Gesamtvolumens des mindestens einen Verdünnungsmittelprodukts durch Subtrahieren des Gesamtvolumens des mindestens einen Arzneimittelprodukts von dem Gesamtvolumen der zusammengesetzten Medikamentendosen, und
Aktualisieren mindestens einiger der Betriebsvorgänge basierend auf dem Gesamtvolumen des mindestens einen Arzneimittelprodukts, dem Gesamtvolumen der zusammengesetzten Medikamentendosen und dem Gesamtvolumen des mindestens einen Verdünnungsmittelprodukts, und
einen Batch-Workflow unter Verwendung des modifizierten Dosisherstellungsprotokolls erstellt;
eine Anwendung (114) für Apotheken-Workflow-Management des lokalen Systems (110), wobei die Anwendung für Apotheken-Workflow-Management so ausgestaltet ist, dass sie
den Batch-Workflow und die Batch-Bestellung von dem lokalen System (110) empfängt,
mindestens eine grafische Benutzeroberfläche veranlasst, einen ersten Verifizierungsschritt für das Gesamtvolumen des mindestens einen Arzneimittelprodukts anzuzeigen,
die mindestens eine grafische Benutzeroberfläche veranlasst, einen zweiten Verifizierungsschritt für das Gesamtvolumen des mindestens einen Verdünnungsmittelprodukts anzuzeigen,
die mindestens eine grafische Benutzeroberfläche veranlasst, einen dritten Verifizierungsschritt für das Gesamtvolumen der zusammengesetzten Medikamentendosen anzuzeigen, und
die mindestens eine grafische Benutzeroberfläche veranlasst, einen vierten Verifizierungsschritt anzuzeigen, der erkennen lässt, dass die zusammengesetzten Medikamentendosen in einem separaten Behälter für jede der aggregierten Dosisbestellungsaufzeichnungen bereitgestellt werden.

2. System nach Anspruch 1, wobei die Anwendung für Apotheken-Workflow-Management so ausgestaltet ist, dass sie ein Drucken von Etiketten für die einzelnen Behälter veranlasst.

3. System nach einem beliebigen der Ansprüche 1-2, wobei die Etiketten ein Dosisetikett oder ein In-Bearbeitung-Etikett umfassen.

4. System nach einem beliebigen der Ansprüche 1-3, wobei das mindestens eine Arzneimittelprodukt ein pharmazeutisches Arzneimittel oder einen Medikamentenwirkstoff zur therapeutischen Wirkung umfasst.

5. System nach einem beliebigen der Ansprüche 1-4, wobei jede Dosisbestellungsaufzeichnung ferner eine Konzentration des mindestens einen Arzneimittelprodukts umfasst, und wobei das lokale System (110) so ausgestaltet ist, dass es das Gesamtvolumen des mindestens einen Verdünnungsmittelprodukts unter zusätzlicher Verwendung der Konzentration des mindestens einen Arzneimittelprodukts ermittelt.

## Revendications

1. Système (100) destiné au traitement de lots d'une pluralité de commandes de doses pour la préparation de doses de médication composées correspondantes, le système comportant :
une première base de données (112) stockant une pluralité de protocoles différents de préparation de dose pour des doses de médication composées associées, chacun des protocoles de préparation de dose spécifiant
le nom de la dose de médication composée associée,
au moins un produit de médicament,
au moins un produit de diluant et
des opérations permettant de vérifier l'au moins un produit de médicament, l'au moins un produit de diluant et la dose de médication composée associée ;
une seconde base de données (112) stockant une pluralité d'enregistrements de commande de dose, chaque enregistrement de commande de dose spécifiant au moins
le nom de la dose de médication composée,
un volume de l'au moins un produit de médicament,
un volume final de la dose de médication composée,
un système local (110) couplé en communication à la première base de données et à la seconde base de données, le système local (110) étant configuré pour
déterminer quels enregistrements parmi la pluralité d'enregistrements de commande de dose incluent le même au moins un produit de médicament et le même au moins un produit de diluant,
agréger des enregistrements de commande de dose parmi la pluralité d'enregistrements de commande de dose en tant que commande de lots pour les enregistrements de commande de dose qui incluent le même au moins un produit de médicament et le même au moins un produit de diluant,
sélectionner le protocole de préparation de dose à partir de la première base de données qui est associé aux enregistrements de commande de dose agrégée,
modifier le protocole de préparation de dose sur la base d'une quantité des enregistrements de commande de dose agrégés
en déterminant un volume total de l'au moins un produit de médicament par addition du volume de l'au moins un produit de médicament qui est spécifié par chacun des enregistrements de commande de dose agrégés,
en déterminant un volume total des doses de médication composées par addition du volume de la dose de médication composée qui est spécifiée par chacun des enregistrements de commande de dose agrégés,
en déterminant un volume total de l'au moins un produit de diluant par soustraction du volume total de l'au moins un produit de médicament à partir du volume total des doses de médication composées et
en mettant à jour au moins certaines des opérations sur la base du volume total de l'au moins un produit de médicament, du volume total des doses de médication composées, et du volume total de l'au moins un produit de diluant et
créer un flux de travail de lots à l'aide du protocole de préparation de dose modifié ;
une application de gestion de flux de travail de pharmacie (114) du système local (110), l'application de gestion de flux de travail de pharmacie étant configurée pour
recevoir le flux de travail de lots et la commande de lots à partir du système local (110),
amener l'au moins une interface utilisateur graphique à afficher une première étape de vérification pour le volume total de l'au moins un produit de médicament,
amener l'au moins une interface utilisateur graphique à afficher une deuxième étape de vérification pour le volume total de l'au moins un produit de diluant,
amener l'au moins une interface utilisateur graphique à afficher une troisième étape de vérification pour le volume total des doses de médication composées et
amener l'au moins une interface utilisateur graphique à afficher une quatrième étape de vérification indiquant que les doses de médication composées sont fournies dans un récipient séparé pour chacun des enregistrements de commande de dose agrégés.

2. Système selon la revendication 1, dans lequel l'application de gestion de flux de travail de pharmacie est configurée pour provoquer l'impression d'étiquettes pour les récipients séparés.

3. Système selon l'une quelconque des revendications 1 à 2, dans lequel les étiquettes incluent une étiquette de dose ou une étiquette de travail en cours.

4. Système selon l'une quelconque des revendications 1 à 3, dans lequel l'au moins un produit de médicament inclut un médicament pharmaceutique ou un ingrédient de médication actif pour un effet thérapeutique.

5. Système selon l'une quelconque des revendications 1 à 4, dans lequel chaque enregistrement de commande de dose inclut en outre une concentration de l'au moins un produit de médicament et dans lequel le système local (110) est configuré pour déterminer le volume total de l'au moins un produit de diluant à l'aide, en plus, de la concentration de l'au moins un produit de médicament.
